# EUROPÄISCHE PATENTSCHRIFT

(11) **EP 0 953 150 B1**
(45) Veröffentlichungstag und Bekanntmachung des Hinweises auf die Patenterteilung: **15.01.2003**
(21) Anmeldenummer: 98900040.1
(22) Anmeldetag: 13.01.1998
(51) Int. Cl.: G01N 27/28, G01N 33/18, G01N 27/333, G01N 33/487, G01N 33/49

(54) **MODULARES SENSORSYSTEM FÜR DIE PROZESS-MESSTECHNIK**
MODULAR SENSOR SYSTEM FOR THE INDUSTRIAL PROCESS MEASUREMENT TECHNIQUE
SYSTEME MODULAIRE DE CAPTEURS UTILE POUR LA TECHNIQUE DE MESURE DE PROCESSUS INDUSTRIELS

(30) Priorität: 14.01.1997 CH 6497
(43) Veröffentlichungstag der Anmeldung: 03.11.1999
(73) Patentinhaber: Eidgenössische Technische Hochschule Zürich, 8092 Zürich (CH)
(72) Erfinder: SPICHIGER-KELLER, Ursula, CH-8804 Au (CH); MÜLLER, Jürg, CH-4600 Olten (CH)
(74) Vertreter: Blum, Rudolf Emil Ernst
(86) Internationale Anmeldenummer: IB9800044
(87) Internationale Veröffentlichungsnummer: WO98030892

(56) Entgegenhaltungen:
- EP-A- 0 148 150
- EP-A- 0 335 859
- EP-A- 0 385 964
- EP-A- 0 467 479
- EP-A- 0 509 791
- EP-A- 0 514 575
- EP-A- 0 723 020
- DE-A- 4 114 959

## Beschreibung

### Querverweis auf verwandte Anmeldungen

Diese Anmeldung beansprucht die Priorität der Schweizer Anmeldung 64/97, eingereicht am 14. Januar 1997, deren Offenbarung hier durch Bezugnahme vollständig einverleibt ist.

### Technisches Gebiet

Die vorliegende Erfindung betrifft die Prozess-Messtechnik, insbesonders ein Sensorsystem für die Prozess-Messtechnik, das die kontinuierliche und selektive Erfassung verschiedener Messparameter ermöglicht.

### Stand der Technik

Es sind bereits Sensorsysteme bekannt und im Stand der Technik beschrieben.

Die bekanntesten Systeme für die kontinuierliche on-line Messtechnik sind die *FIA-Systeme* (Flow-Injection-Analysis). Diese Systeme bestehen aus einem aufwendigen Fluidics-System mit Pumpen und Ventilen. Sie verwenden traditionelle analytische Bestimmungsverfahren mit Reagentien und mitunter Biosensoren oder Ionen-selektiven Elektroden (ISE) am Ende des Fliesssystems als Detektoren.

Die bekannten Sensorsysteme sind ausschliesslich definiert und spezifiziert durch die Verwendung entweder der potentiometrischen oder der voltammetrischen (elektrochemische Messung der Aenderung des Sauerstoff-Partialdruckes) oder der amperometrischen oder der optischen Messtechnik. Die Firma YSI (2700 SELECT) bietet ein Messgerät mit Biosensoren für die kontinuierliche Prozessmesstechnik an. Die Sensorelemente stecken in einer miniaturisierten Messkammer, in welche das Untersuchungsmaterial eingefüllt und daraus wieder abgesaugt wird. Die amperometrischen oder voltammetrischen Systeme umfassen die Biosensoren sowie miniaturisierte modifizierte Elektroden wie "glassy carbon" oder "carbon paste" Graphit-Elektroden [vgl. Kalcher, K.; Kauffmann, J.-M.; Wang, J.; Svancara I.; Vytras, K; Neuhold, C.; Yang, Z. Electroanalysis 7/1 (1995) 5-22 (Review)]. Veröffentlichungen wurden insbesonders bezüglich Arrays (Serieschaltung von mehreren vom Funktionsprinzip her identischen Sensoren auf einer geometrisch definierten Sensorfläche) gemacht unter Verwendung von miniaturisierten Sensoren, sog. *solid-contact* Elektroden bzw. *ISFETs* (ion-selective fieldeffect transistors)[vgl. Schindler, J.G.; Schindler, M.M.; Herna, K; Reisinger, E.; Kulmann, U.; Graef, R.; Lange, H. *Biomed Tech* 36/11 (1991) 271-280, 282-284. (HCA 116:136186.); van der Schoot, B.H.; Jeanneret, S.; van den Berg, A.; de Rooij, N.F. *Sensors and Actuators B* 15/1-3 (1993) 211-213; Hoffmann, W.; Rapp, R.; Ache, H.J.; Stolze, D.; Neuhaus, D.; Hofmann, D.; Freywald, K.H. Micro Total Anal. Syst., Proc. µ-TAS'94 Workshop 1st (1995). in van den Berg, A.; Bergveld, P. (Eds). Dordrecht: Kluwer, Netherlands]. Diesen Systemen fehlt einerseits die Robustheit (ruggedness), insbesondere der Schichtstruktur und des Referenzsystems, andererseits die Flexibilität, um auf Neuentwicklungen reagieren zu können. Abhängig von der Fertigungstechnologie, sind sie zudem nur für eine begrenzte Auswahl resp. Typen selektiver Schichten optimierbar. Damit sind ihre Anwendungsmöglichkeiten eng begrenzt. Aenderungen von mikrostrukturierten Sensorplattformen lohnen sich kaum oder nur, wenn extrem grosse Stückzahlen hergestellt werden können. Andererseits werden die miniaturisierten Sensoren aufgrund ihrer unbefriedigenden Robustheit und Langzeitstabilität vorwiegend als "One-Way" Sensoren auf den Markt gebracht (i-STAT). Dies induziert die Produktion von Abfall von kostbaren Materialien.

Kontinuierliche Messysteme brauchen im allgemeinen die Möglichkeit einer Rekalibration um eine hohe Zuverlässigkeit der Resultate zu erzeugen. Allerdings wurden auch kalibrationsfreie Systeme bereits untersucht [Rumpf, G.; Spichiger, U.E.; Bühler, H.; Simon, W.; *Anal. Sciences* 8 (1992) 553-559)].

Die selektive Bestimmung bestimmter Bestandteile eines Gemisches der Substanz ist nur möglich durch Anwendung sogenannter Selektivitätsprinzipien.

Eine grosse Zahl von Selektivitätsprinzipien auf der Basis von Erkennungsmolekülen, z.B. selektiven Liganden, oder wirksamen Selektivitätsprinzipien, welche auf Verteilungsgleichgewichten beruhen, sind bekannt [Fluka-Katalog "Selectophore®", 1996]. Einzelne dieser Selektivitätsprinzipien wie z.B. die Magnesium-, Nitrit-, Schwermetall-selektiven Liganden und Schichten wurden massgeblich weiter entwickelt [Schaller, U.; Bakker, E.; Spichiger, U.E.; Pretsch, E. *Talanta* 41/6 (1994) 1001-1005; Spichiger, U.E.; Eugster, R.; Citterio, D.; Li, H.; Schmid, A.; Simon, W. In: S. Golf, D. Dralle, L. Vecchiet (Eds) "Magnesium 1993" London: John Libbey & Comp., 1993].

Substanz-selektive Liganden werden im Bulk einer Schicht gelöst. Diese Schicht oder Membran liefert oft erst nach sorgfältiger Optimierung des Bulkmediums, beispielsweise der Bulkzusammensetzung, eine befriedigende Selektivität. Wird eine solche Schicht in vorbestehende Sensorelemente eingearbeitet durch Eingiessen, Einkleben, Photopolymerisation, u.a Polymerisationsverfahren muss oft mit einem beträchtlichen Verlust der Selektivität bzw. der Sensitivität Vorlieb genommen werden.

Die Verfahrenstechnik zum Aufbringen von Schichten und zur Immobilisierung von Selektivitätsprinzipien mittels Photopolymerisation grenzt die Möglichkeiten der Anwendung selektiver Schichten stark ein und schöpft das Potential der Erkennungskomponenten nicht aus.

Trotz der überaus grossen Anzahl von bekannten Liganden und Erkennungskomponenten wurden bisher nur wenige in eine Schicht eingebracht und für analytische Anwendungen getestet. Damit wurden bisher auch nur vereinzelte Liganden in Chemischen Sensoren verwertet. Die Verwendung von Chemischen Sensoren in Messinstrumenten hat ausschliesslich in der Medizintechnik seit Jahren einen festen Platz. Elektrolyte werden in Automaten für die tägliche Routine, aber auch in "bed-side"-Analysatoren oder Geräten für die Arztpraxis unter Verwendung von Ionen-selektiven Sensoren bestimmt. Das erste Gerät, das sich optische Sensoren zu Nutze macht ist auf dem Markt (*OPTI,* AVL Biosense Corp., Atlanta). Das Gerät arbeitet mit "disposable cartridges" und bietet die Parameter pO₂, pCO₂ und pH an (Fluoreszenzemissionsmessung). Einzelne Geräte haben seit kurzem eine Kombination von Biosensoren (voltammetrische resp. amperometrische Bestimmung der Aenderung des Sauerstoff-Partialdruckes, z.B. nach Olsson, L. et al., Anal.Chim. Acta 224(1) (1989) 31 ff) für Laktat, Glucose, Peroxid, Glutamat, Kreatinin zusammen mit Ionen-selektiven Elektroden implementiert. Diese Kombinationen beruhen auf miniaturisierten Sensoren auf der Basis von Feststoffableitungen oder Halbleiterelementen vom Typ ISFET / ENFET (Ionen-selektive-, enzymatische Feldeffekt-Transistoren), bei welchen die Leitfähigkeits- bzw. Potentialänderung über dem Halbleiterelement detektiert wird. Eine solche Kombination hat die Firma ISTAT als "disposable sensors" für den Einmal-Gebrauch auf den Markt gebracht. Diese Sensoren weisen die bereits oben beschriebenen Nachteile auf, u.a. mangelnde Robustheit und grosse Abfallmengen.

Eine Firma, die seit vielen Jahren einen amperometrischen Glucosesensor ExacTech® für den Selbsttest verkauft, ist die MediSense Inc. (Waltham, MA) mit einem Sitz in Basel; ein entsprechendes Gerät für die industrielle Anwendung ist der YSI Glucose-Sensor von Yellow Springs (OH, USA). Die in diesen Geräten eingesetzten Sensoren weisen mehrere Schichten auf, welche letztlich zur Selektivität der Biosensoren führen. Jeder angebotene Biosensor beruht auf demselben Elektrodenprinzip, welche eine Messung des Sauerstoffverbrauchs mit der herkömmlichen elektrochemischen Sauerstoffelektrode bestimmt.

Seit 1988 wurden mehr als 1000 Publikationen über Glucose-Biosensoren veröffentlicht (vgl. Gorton, L. Electroanalysis 7/1 (1995) 23-44.) Dagegen sind optische Sensoren noch kaum eingeführt. WPIDS (World Patents Index No) 95-256069 [34] beschreibt die optische Messung von Streulicht in lebenden Geweben und WPIDS 93-213750 [26] die optische Messung des Blut-Haemoglobingehaltes (measuring of blood hemoglobin content).

Ferner offenbart die Patentveröffentlichung EP-A-0 723 020 ein Modul gemäß Oberbegriff des unabhängigen Anspruchs 11.

Ziel der vorliegenden Erfindung war es deshalb, ein Messystem, insbesonders für die kontinuierliche Prozess-Messtechnik, bereitzustellen, das die Nachteile bekannter Sensorsysteme und Sensoren nicht aufweist und sich insbesonders durch grosse Flexibilität und Robustheit auszeichnet.

### Darstellung der Erfindung

Das angestrebte Ziel wurde erreicht durch die Bereitstellung eines kontinuierlichen, modulären, insbesonders mehrdimensionalen, und robusten Messystems, das idealerweise reagenzienfrei betrieben wird, sowie die Bereitstellung spezieller modularer Sensorelemente und für solche Sensorelemente geeignete Erkennungssysteme.

Zum besseren Verständnis der Erfindung werden vorab einige grundlegende Begriffe definiert:

### - Mehrdimensionales (multidimensionales), modulares, kontinuierliches Messsystem; Sensorelement; Halbzelle; Messmodul; Referenz; Referenzmodul

Ein kontinuierliches Messsystem auf der Basis von Sensorelementen setzt voraus, dass diese Sensoren reversibel oder innert der gewünschten Reaktionszeit regenerierbar arbeiten, d.h. dass sich ihr Signal resp. die generierte Messgrösse immer wieder den kurzfristig wechselnden Eigenschaften des Specimens anpasst. Das Messsystem kann sowohl im Batchverfahren wie im Durchfluss die kontinuierliche Messung ermöglichen. Der Begriff *modulär* meint, dass die einzelnen Sensoren und Sensorgruppen/-typen/-verfahren als Sensorelemente in einzelne Module implementiert sind und als solche kombiniert werden können. Die unterschiedlichen Sensorelemente können beispielsweise in stabförmige Module, z.B. für die Reaktortechnik, implementiert werden. Solche Module sind insbesonders für den Durchfluss geeignet. Charakteristischerweise können durch geeignete Dimensionen dieser Module auch verschiedene Messverfahren im selben Messsystem miteinander kombiniert werden. Dadurch wird das System *multidimensional,* da die *Information* über die *Substanz* aufgrund voneinander unabhängiger Messverfahren gewonnen werden kann. Mehrdimensional (multidimensional) bezeichnet die Eigenschaft des Messsystems, dass mehrere Sensoren bzw. Sensorelemente oder Messverfahren kombiniert und damit mehrere Substanzen in Serie oder parallel bestimmt werden können. Zwei Verfahren, d.h. zwei verschiedene Typen von Modulen können auch unabhängige, d.h. orthogonale Informationen über dieselbe Substanz liefern und damit die Betriebssicherheit erhöhen (z.B. ein optisches und ein potentiometrisches oder ein optisches und eine amperometrisches Modul). Ein Modul kann auch nur eine Halbzelle bzw. ein Referenzelement eines Sensorelementes enthalten oder auch mehr als ein Sensor- oder Ableitelement. In der Folge wird - wo eine Spezifizierung notwendig ist - ein Modul, das der Messung selbst dient, sei es eine Halbzelle oder ein vollständiges Element als Messmodul bezeichnet, währenddem das Referenzelement resp. die Referenzhalbzelle als Referenzmodul bezeichnet wird.

### - Robustheit

Unter Robustheit (ruggedness) versteht man allgemein die geringe Abhängigkeit der generierten Messgrösse von kleinen Aenderungen der Parameter des Messsystems bzw. die geringe Störanfälligkeit einer Messtechnik bzw. des Messsystems, worunter auch die Langzeitstabilität gezählt werden kann. Die Robustheit kann durch Automatisierung der Messtechnik und durch automatisierte Rekalibrierung zusätzlich verbessert werden.

### - Substanz, Zielsubstanz, Analyt; Gruppe von Zielsubstanzen, Begleitsubstanzen,

Als Zielsubstanz oder Analyt (target compound, target analyte) wird die Substanz oder eine Substanzgruppe/Substanzklasse bezeichnet über die eine (physikalisch-chemische) Information gewonnen werden soll. Diese Information wird in der Regel der Quantifizierung der Substanz in ihrem Medium dienen und eine quantitative Information liefern, auf welche eine Entscheidung (decision making) folgt. Das chemische Sensorelement oder eine Gruppe von Sensorelementen ist in der Lage zwischen der Zielsubstanz resp. einer Gruppe von Zielsubstanzen und Begleitsubstanzen zu unterscheiden und die Begleitsubstanzen zu diskriminieren.

### - Selektive Schichtstruktur; Selektivitätsprinzip

In der Schichtstruktur bilden das Selektivitätsprinizip bzw. der Erkennungsschritt und der Transduktionsschritt (vgl. unten) eine aufeinanderfolgende Einheit. Die Schichtstruktur kann aus einer einzigen Schicht oder mehreren Schichten, einschliesslich Hilfsschichten mit definierten Funktionen, bestehen. Man spricht von einer selektiven Schichtstruktur.

### - selektive Erkennungsvorrichtung; Selektivitätsprinzip bestehend aus: Erkennungsschritt; Verteilungsgleichgewicht; Partition; Erkennungskomponente; reagentienfrei

Der Erkennungsschritt führt zur typischen Selektivität jedes einzelnen Sensors bzw. Sensormoduls. Als Folge des Selektivitätsprinzips wird die Zielsubstanz gegenüber Begleitsubstanzen vorgezogen. Das Selektivitätsprinzip kann auf Verteilungsgleichgewichten/Partition und/oder typischen chemischen Wechselwirkungen zwischen Zielsubstanz und mindestens einer Erkennungskomponente beruhen. Die Schichtstruktur enthält dabei die massgeblichen Komponenten der chemischen Erkennung. Das Selektivitätsprinzip ist in der Regel reversibel oder dem Zeittakt des kontinuierlichen Messystems entsprechend regenerierbar. Die Kombination dieser Schritte kann als selektive Erkennungsvorrichtung bezeichnet werden.

### - Transduktionsschritt

Als Transduktionsschritt wird ein Schritt oder eine Folge von Schritten bezeichnet, welche(r), induziert resp. ausgelöst durch den selektiven Erkennungschritt bzw. das Selektivitätsprinzip, die Bildung einer in der Regel quantifizierbaren Messgrösse bewirkt.

### - Messgrösse

Die Bildung der Messgrösse ist das Resultat aus dem Erkennungsschritt mit seinem typischen Selektivitätsprinzip und dem darauf folgenden Transduktionsschritt. Die Messgrösse kann sich bereits als Folge des aufgrund des Selektivitätsprinzips veränderten Verteilungsgleichgewichtes bilden (Potentiometrie, IR-Spektroskopie) oder durch Ankopplung von Hilfsstoffen (z.B. Indikatoren) an den Erkennungsschritt.

### - Information

Die Messgrösse führt nach der Ableitung und ev. Verstärkung auf ein Datenaufnahme- und -verarbeitungssystem zur Bildung einer Information über die Zielsubstanz oder eine Gruppe von Zielsubstanzen. Die Messgrösse muss, um eine höhere Sicherheit der Aussage zu erhalten, oft mit einer Referenzgrösse abgeglichen werden. Die Information kann in einer qualitativen meist aber quantitativen Information bestehen, aber auch Bewertungs- bzw. Klassifizierungskriterien enthalten.

Die oben definierten Begriffe werden in der Folge in ihrer angegebenen Bedeutung verwendet.

Das erfindungsgemässe modulare System zur kontinuierlichen, reagentienfreien Bestimmung einer Substanz ist dadurch gekennzeichnet, dass es mindestens zwei Messmodule gleichen oder verschiedenen Modultyps zur simultanen Gewinnung von mindestens zwei Informationen aufweist, wobei jedes Modul mindestens eine auswechselbare selektive Schichtstruktur und eine Ableitvorrichtung enthält, wobei die Schichtstruktur einen selektiven Erkennungsschritt ermöglicht und eine kontinuierliche Messung zulässt. In einer bevorzugten Ausführungsform des Messystems werden verschiedene Modultypen kombiniert, z.B. mindestens ein ionen-selektives Modul und mindestens ein Modul eines anderen Modultyps etc. Bei 3 Modulen kann 1 Modul ein Referenzsystem enthalten. Das Referenzsystem unterscheidet sich vom Messmodul dadurch, dass es kein Selektivitätsprinzip enthält oder dass das Selektivitätsprinzip nicht aktiv ist.

Weitere Gegenstände der vorliegenden Erfindung sind ein Verfahren zur kontinuierlichen Bestimmung einer Substanz, die Verwendung des Systems sowie Module und Erkennungskomponenten, die für die Verwendung im Messystem geeignet sind. Spezielle Ausführungsformen sind in den Unteransprüchen beschrieben.

Reversible und auch schnell regenerierbare chemische Sensoren zeichnen sich durch die Möglichkeit der *kontinuierlichen Messung* einer Zielkomponente, der *Substanz,* aus. Zur Gewinnung von kontinuierlichen Informationen über ein breites Spektrum von Substanzen ist nur die FIA (Flow injection analysis) als vergleichbare Messtechnik zu nennen. Im Gegensatz zu Chemischen Sensoren, werden bei der FIA traditionelle chemische Reaktionen unter Verwendung von Reagentien eingesetzt. Das Verfahren zeichnet sich durch ein hoch entwickeltes aber kostenintensives Fluidics-System aus.

Das erfindungsgemässe Messystem zeichnet sich im Gegensatz zu bestehenden Messsystemen durch maximale Flexibilität, Reagentienfreiheit und Robustheit gegenüber der Beschaffenheit des Untersuchungsmaterials und gegenüber der kontinuierlichen Langzeit-Messtechnik aus. Daher kann es auch für die Bestimmung von Gasen und flüchtigen Substanzen verwendet werden. Durch Automatisierung lassen sich die erfindungsgemässen Vorteile weiter verbessern. Die Flexibilität wird erreicht, indem im Prinzip alle bekannten Sensortypen implementierbar sind; insbesonders können z.B. potentiometrische und/oder voltammetrische Messtechniken mit mindestens einer anderen Messtechnik kombiniert werden, beispielsweise der amperometrischen und/oder der optischen. Die Kombination solcher verschiedener Messtechniken ermöglicht die Bereitstellung breitester Information über eine oder mehrere Zielsubstanzen. Ferner erhält man über die Kombination solcher verschiedener Messtechniken für die Messung der gleichen Zielsubstanz die grösstmögliche Gewähr für korrekte Messergebnisse, insbesonders bei kalibrationsarmen resp. -freien Systemen. Die selektive Schichtstruktur, die für jedes Modul charakteristisch ist und in der vorzugsweise der Erkennungs- *und* der Transduktionsschritt abläuft, bildet das Herzstück des Sensorelementes. Die Robustheit des Sensorelements wird durch die Optimierung der Schichtstruktur, durch die Referenzbildung, die Automatisierung, durch den Bau des Moduls und den modulären Aufbau gewährleistet.

Ein wesentliches Merkmal des Systems ist deshalb, dass das Selektivitätsprinzip, die selektive Schichtstruktur, austauschbar ist; sie ist unter einer Vielzahl von verfügbaren Schichten austauschbar und, entsprechend einer beschränkten Lebensdauer, auswechselbar. Das Selektivitätsprinzip, die selektive Schichtstruktur, kann auf einem Träger aufgebracht verpackt und in veschiedener Grösse gehandelt werden. Ein solcher Träger kann bereits im Ableitsystem bestehen (z.B. planarer Wellenleiter, planare oder Stab-Elektrode, optische Faser, Resonator, SAW-Chip). Das austauschbare Selektivitätsprinzip kann dann als chemisch-aktiver Chip bezeichnet werden. Der chemisch-aktive Chip oder Chemochip ist ein wesentliches Element des Messystems. Ferner gehört zu den wesentlichen Merkmalen, dass nicht nur verschiedene Sensoren sondern auch verschiedene Typen von Sensoren als selbständige Module in Serie oder in beliebiger anderer Anordnung miteinander verbunden werden. Die Schaltung in Serie ist für die Messung im Durchfluss bevorzugt (optimaler Durchsatz von Specimen). Parallelschaltung ist für die Messung von Untersuchungsmaterial, das unterschiedlich konditioniert werden muss (pH, Ionenstärke, Temperatur etc.) bevorzugt.

Ein Modul enthält alle für das Selektivitätsprinzip, den Erkennungsschritt, den Transduktionsschritt, die Bildung der Messgrösse und die Ableitung notwendigen Elemente. Es ist infolge seiner Robustheit mehrfach resp. kontinuierlich über längere Zeit verwendbar. Das Selektivitätsprinzip, die Analyt-selektive Schicht, kann aufgrund der speziellen Art der Implementierung, z.B. für die sterile oder aseptische Messtechnik von der Seite, wo die Ableitung eingebracht wird, kalibriert werden. Dies ist deshalb möglich, weil das Ableitelement nicht zwingend untrennbar mit der selektiven Schichtstruktur verbunden ist.

Das einzelne Modul arbeitet reagentienfrei. Unter Reagentienfreiheit versteht man, dass kein Hilfsstoff als Reagens verwendet wird, der die chemische Erkennungsreaktion zusätzlich zum Selektivitätsprinzip ermöglicht. Die erfindungsgemässen Module unterscheiden sich damit stark von der FIA-Technik und verwandten kontinuierlichen Messystemen. Eine Pufferung, Zusatz von Co-Substrat oder weitere Konditioniermittel werden nicht als Reagentien bezeichnet und deshalb durch den Begriff Reagentienfreiheit nicht ausgeschlossen.

Ein einzelnes Modul kann auch nur aus einer Halbzelle bestehen, ein anderes kann dann das Referenzsystem sein, das durch seine (Puffer)-Kapazität ebenfalls robust und entsprechend langzeitstabil ist. Die Vereinigung verschiedener selbstständiger Sensormodule mit verschiedenen Funktionsprinzipien zu einem Messsystem wird als *multidimensionales oder mehrdimensionales System* bezeichnet. Jedes Modul kann unabhängig vom anderen sein und durch einen orthogonalen Beitrag optimal zur Endinformation über die Substanz/-en beitragen. Die Ernte für den Benutzer besteht darin, dass er nicht mehr selektiv die Messtechnik auszuwählen braucht, sondern lediglich bestimmt, über welche analytischen Parameter bzw. Zielsubstanzen (target compounds) er Informationen braucht und welche analytische Spezifikation (analytical performance) er erfüllt wissen will. Er kann dann die Entscheidung, welche Messtechniken, sprich Sensormodule, unter diesen Umständen vorzugsweise zu kombinieren sind, beispielsweise dem Anbieter überlassen. Dasselbe gilt für die Entscheidung, wieviele Messtechniken und Module zu kombinieren sind, um die vorgegebenen Spezifikationen bzw. bestmögliche Betriebssicherheit zu erreichen.

Erfahrungen mit verschiedenen Sensortechnologien zeigen, dass verschiedene Prinzipien verschiedene Eigenschaften aufweisen, die mehr oder weniger geeignet sein können, dass aber keine Basistechnologie alleine den optimalen Betrieb für verschiedene Anwendungen abdecken kann. So wurde beispielsweise gefunden, dass sich für die Messung des gelösten Sauerstoffes (des O₂-Partialdrucks in Lösung) die optische Messtechnik durch ihre Einfachheit und das schnelle Ansprechverhalten auszeichnet und damit für modulare Sensoren resp. Sensorsysteme gemäss der vorliegenden Erfindung speziell geeignet ist. Die optische Messtechnik ist diesbezüglich eine gute Alternative zu elektrochemischen Sensoren. Die optische Messtechnik ist auch möglich für Messungen der Natrium-, Kalium- und Calcium-, Nitrit- und Chloridaktivität sowie zahlreicher ungeladener Zielkomponenten und Substrate wie Alkohole, Amine, Glucose, Feuchtigkeit, Laktat, etc. Gase, wie NOₓ, SO₂, NH₃ u.a. direkt im Specimen.

Speziell geeignete kalibrationsfreie Assays sind beispielsweise die Zerfallszeit (luminescence decaytime) wie in Draxler, S.; Lippitsch, M.E.; Klimant, I.; Kraus, H.; Wolfbeis, O.; J.Phys.Chem. 99/10 (1995) 3162-3167 beschrieben und die symmetrische Messtechnik: G. Rumpf; U. Spichiger-Keller; H. Bühler und W. Simon; Calibration-Free Measurement of Sodium and Potassium in Undiluted Human Serum with an Electrically Symmetric Measuring System. *Anal.Sciences* 8 (1992), 553-559. Die symmetrische Messtechnik z.B. nach Haase, A.E.; *Untersuchung der Wechselwirkung zwischen ionenselektiven Flüssigmembranen und Messgut im Hinblick auf die kontinuierliche Erfassung von Kationen im Blut.* Diss ETH Nr. 10453 (1993) eignet sich zur Herstellung von Referenzmaterialien für die Qualitätssicherung der elektrochemischen Bestimmung der aktiven Molalität von Ionen. Solche Referenzmaterialien/Qualitätskontrollproben sind zur Zeit weltweit nicht erhältlich.

Es wurde nun gefunden, dass mittels einer solchen symmetrischen Messtechnik resp. durch Verwendung von symmetrischen Membranen, welche mit dem Specimen optimal kompatibel sind, und einer symmetrischen Anordnung/Konfiguration der Messzelle Referenzmaterialien für die Bestimmung der molalen Aktivität von Ionen beispielsweise zur Qualitätsstcherung herstellbar sind. Als Referenzmaterial kommen beispielsweise wässrige Lösungen aus lyophilisiertem Blutplasma oder -serum in Frage.

Insbesondere für Messungen im Pharma- resp. Medizinal- und Lebensmittelbereich ist es notwendig resp. sinnvoll, die Module zu sterilisieren. Diesbezüglich kann auf gängige Verfahren des Stands der Technik verwiesen werden, z.B. für die Sterilisation auf Haase, A.E.; *Untersuchung der Wechselwirkung zwischen ionenselektiven Flüssigmembranen und Messgut im Hinblick auf die kontinuierliche Erfassung von Kationen im Blut.* Diss ETH Nr. 10453 (1993), S. 82-83. γ-Strahlen Sterilisation für verpackte Membranen ist möglich. Auch ist es notwendig die Einführung toxischer Komponenten in die gemessene Probe, das Specimen, zu vermeiden. Toxizität von ISEs wird z.B. beschrieben in Haase, A.E.; *Untersuchung der Wechselwirkung zwischen ionenselektiven Flüssigmembranen und Messgut im Hinblick auf die kontinuierliche Erfassung von Kationen im Blut.* Diss ETH Nr. 10453 (1993). Amperometrische Systeme auf der Basis der Mediatoren TTF/TCNQ (Tetrathiafulvalen-p-tetracyanochinondimethan) gelten allgemein toxikologisch als unbedenklich.

### Kurze Beschreibung der Zeichnungen

Figur 1 stellt ein erfindungsgemässes moduläres dreidimensionales System für die kontinuierliche Prozessmesstechnik mit einem potentiometrischen, einem amperometrischen und einem optischen selektiven Sensorelement im Längsschnitt dar.
Figur 2 zeigt einen Schnitt senkrecht zur Längsachse des Systems von Figur 1 durch ein potentiometrisches Element mit austauschbarer Ionen-selektiver Membran,
Figur 3 zeigt einen Schnitt senkrecht zur Längsachse des Systems von Figur 1 durch ein erfindungsgemässes amperometrisches Modul mit Biosensor-Element,
Figur 4 zeigt einen Schnitt senkrecht zur Längsachse des Systems von Figur 1 durch ein erfindungsgemässes optisches Modul mit faseroptischem Element,
Figur 5 zeigt einen Schnitt senkrecht zur Längsachse eines Systems entsprechend Figur 1 durch ein erfindungsgemässes optisches Modul mit Diodenelement,
Figur 6A und 6B stellt den Reaktionsweg der Hypoxanthinanalyse dar, wobei

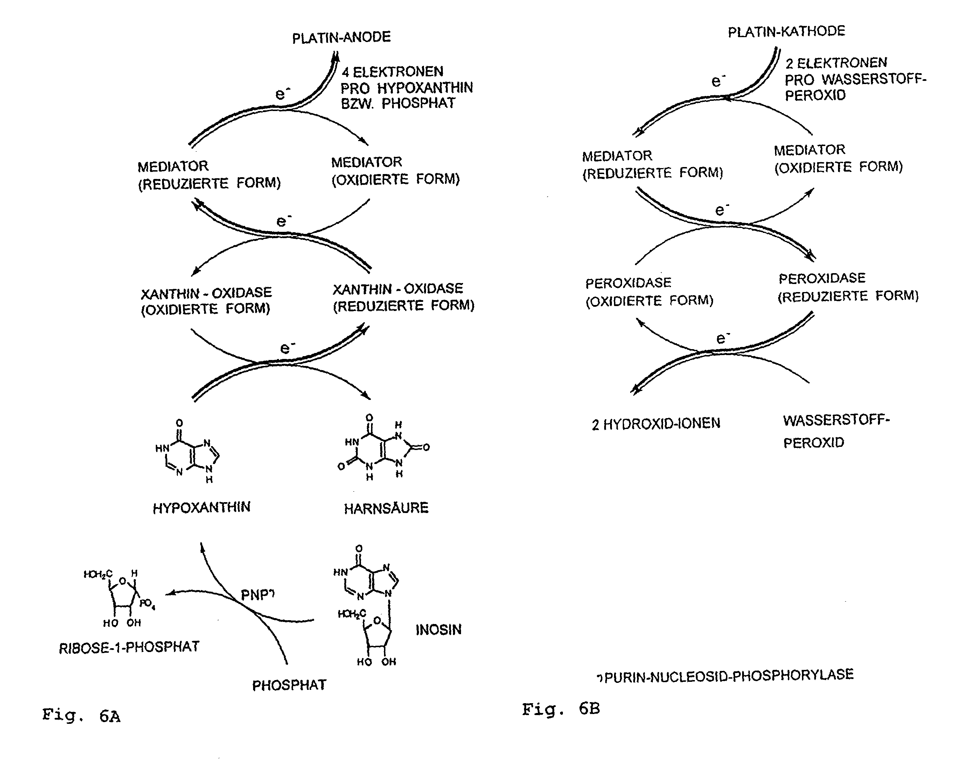

Figur 6A den Oxidationsschritt zeigt und
Figur 6B den Reduktionsschritt.

Die Bezugszeichen in den Figuren haben die nachfolgend angegebene Bedeutung:
I: Potentiometrisches Modul (Halbzelle), Ionen-selektives Sensorelement
II: Amperometrisches Modul (Halbzelle), Biosensor-Modul
III: Optisches Modul; Faseroptisches Modul für die Fluoreszenz- oder Reflektionsmessung.

1 Träger des Durchflusssystems (z.B. aus Aluminium)
2,3,4 Körper der Elektrodensegmente des Durchflussystems (z.B. aus Polymethylmethacrylaten)
   dabei bezeichnet
2 Segment der Referenz-Elektrode, unterer Teil
3 Segment der Messelektrode, Basisteil
4 Körper der Messelektrode, Kopfteil
5,6 Schrauben für die dichte Fixierung der Module
7 Durchflusskanal für das thermostatisierte Wasser
8 Selektive Schichtstruktur
9 Probekanal
10 Thermoelement
11 Kapillare mit innerem Elektrolyt
12 Ableitvorrichtung/Ableitelement
13 Referenzelektrode/Referenzmodul (für potentiometrische/amperometrische Halbzelle)
14 Körper des Ableitelements/der Ableitvorrichtung
15 Schraube für eine dichte Verbindung zwischen der Membrane/selektiven Schichtstruktur und dem Probekanal
16 Detektionsdiode (auf Platte)
17 Emissionsdiode

### Weg zur Ausführung der Erfindung

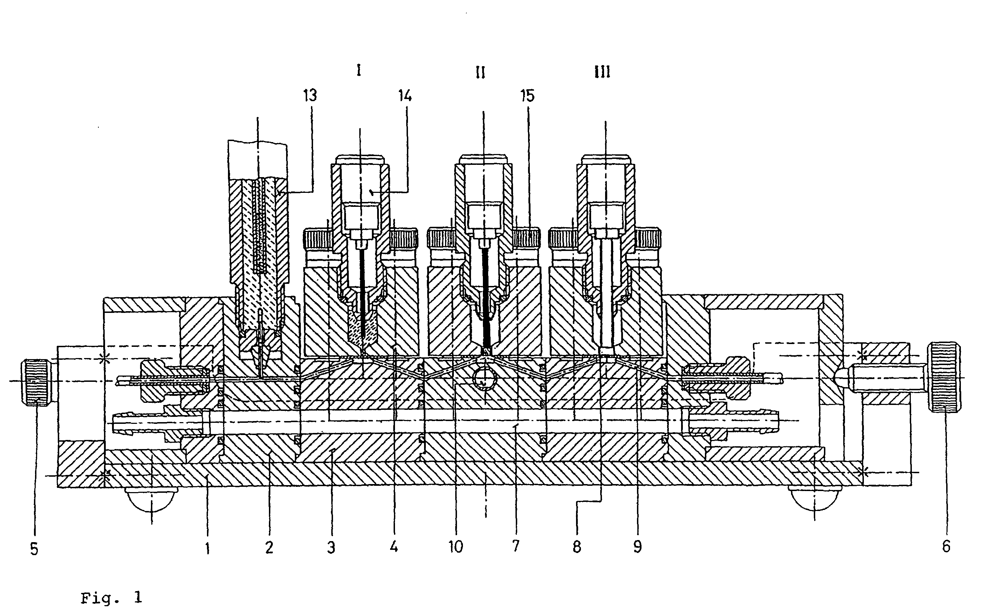

Das einzelne Modul I, II, III, 13, das für die Verwendung in dem erfindungsgemässen System geeignet ist, besteht aus dem Körper des Moduls 3, 4, der aus z.B. Kunststoff gefertigt wird (siehe Figur 1).

Der Körper umfasst eine austauschbare selektive Schichtstruktur 8 und eine Ableitvorrichtung 12 sowie vorzugsweise einen Kanal 9 bzw. einen Schlauch für den kontinuierlichen bzw. Stop-Flow Transport von Probe oder Specimen und für die kontinuierliche Messung. Der Körper umfasst somit
das Sensorfeld, wo die selektive Schichtstruktur bzw. die selektive Erkennungsvorrichtung 8 (z.B. Ionenselektive Membran; Enzymaktive Paste, Optodenmembran, beschichteter Wellenleiter, IR-Wellenleiter) einerseits mit der Probe andererseits mit der Ableitvorrichtung 12 in Verbindung steht und wo der Erkennungsschritt stattfindet,
den Transduktionsschritt, wo der Erkennungsschritt in eine Messgrösse umgesetzt wird, die dann über die Ableitvorrichtung 12 (z.B. innere Elektrode, optische Faser, Detektordiode, Verstärker und Draht für die Ableitung des elektrischen Stroms) abgeleitet und fakultativ mit einer Referenzgrösse verglichen wird.

Das Modul kann in der zweckmässigen Grösse hergestellt, vorzugsweise im Centimeter bis Mikrometer Massstab angefertigt werden. Das einzelne Modul kann ein selbständiges chemisches Sensorelement bilden oder in einer Halbzelle (z.B. Potentiometrie) bestehen, wobei dann vorzugsweise ein zweites Modul die andere Halbzelle enthält. Module können nach Bedarf und in beliebiger Anordnung miteinander verbunden werden (z.B. in Serie oder parallel geschalten). Verschiedene Module unterscheiden sich durch den Erkennungs- und/oder Transduktionsschritt, für den ein Modul ausgelegt ist und durch den ein Modul sich vom andern unterscheidet. Module mit demselben Transduktionsprinzip und daher derselben Bauart werden zu Modultypen/Modulklassen zusammengefasst.

Modultypen, welche sich durch verschiedene Messprinzipien unterscheiden und damit verschiedene, voneinander unabhängige Dimensionen der Information über die Substanz liefern (aber nach demselben Grundbauprinzip realisiert sind), sind z.B.:
- potentiometrische Halbzelle mit ionenselektiver Membran oder kombiniertes potentiometrisches Ionen-selektives Elektrodenelement
- selektives amperometrisches Biosensorelement, z.B. mit selektiver leitender Schicht/Paste
- Element mit selektivem Erkennungsschritt und einem beschichteten oder unbeschichtetem optischen planaren Wellenleiter als Transduktions- und/oder Ableitelement
- Element mit selektivem Erkennungsschritt und Ableitelement für die Absorptions-, Emissions- bzw. Reflektionsmessung mit beschichteter oder unbeschichteter optischer Faser
- selektives "Surface Acoustic Wave"-Sensorelement
- konduktometrisches Sensorelement mit Selektivitätsprinzip
- kapazitives Sensorelement kombiniert mit einem Erkennungsschritt
- optisches oder elektrochemisches Referenzelement/Halbzelle.

Module für die Potentiometrie sind bereits bekannt, doch wurde der Vorteil einer austauschbaren Schichtstruktur noch nicht erfasst. Es wurde nun gefunden, dass potentiometrische aber insbesonders auch amperometrische und optische Module mit auswechselbarer Schichtstruktur hergestellt und für die kontinuierliche Messtechnik eingesetzt werden und fest fixierten Schichten gegenüber überlegen sein können.

Wesentlicher Bestandteil jeden Moduls ist die selektive Schichtstruktur.

Diese umfasst mindestens eine Komponente, welche die Selektivität des Sensorelementes bewirkt, d.h. die bevorzugte Erfassung der Zielsubstanz bzw. einer Gruppe von Zielsubstanzen gegenüber Begleitsubstanzen erlaubt. Die Selektivität kann durch molekulare Erkennung der Zielsubstanz bewirkt werden oder durch die Partition der Substanz zwischen Probe/Specimen und dem Sensorelement (durch ein Verteilungsgleichgewicht). Die selektive Schichtstruktur kann Hilfsstoffe enthalten, welche zur Bildung der Schicht nötig sind und/oder den Erkennungsschritt unterstützen, katalysieren und/oder den Einfluss von Interferenzen vermindern, sowie fakultativ eine weitere Komponente, welche für den Transduktionsschritt verantwortlich ist. Die Schichtstruktur umfasst die Möglichkeit die selektive Schicht mit Hilfsschichten zu kombinieren, welche z.B. die Biokompatibilität gewährleisten [vgl. Wintermantel, E.; Ha S-W. (Eds.); Biokompatible Werkstoffe und Bauweisen, Berlin: Springer-Verlag, 1996] und/oder die Trennung zwischen gasförmigen, neutralen und geladenen Substanzen ermöglichen und/oder eine Diffusionsbarriere bilden.

Die Schichtstruktur kann sowohl mehr oder weniger lipophile/apolare wie mehr oder weniger hydrophile/polare Schichten wie auch Mizellen oder Umkehrmizellen enthalten. Solche Schichtstrukturen für optische Sensorelemente wurden bereits in Vaillo, E.; Walde, P.; Spichiger, U.E.; Analytical Methods and Instrumentation, AMI, 2/3 (1995) 145-153 beschrieben. Das Auswaschen von Komponenten in die Probe/das Specimen wird z.B. durch hohe Lipophilie der Komponenten verhindert, im Falle wo eine wässrige Probe / ein wässriges Specimen vorliegt [vgl. Dinten, O.; Spichiger, U.E.; Chaniotakis, N.; Gehrig, P.; Rusterholz, B.; Morf, W.E.; Simon, W. Anal. Chem. 63 (1991) 596-603 für optische und potentiometrische Sensoren] oder durch Immobilisierung einzelner Komponenten.

Die selektive Schichtstruktur wird erfindungsgemäss weder mit selektivitäts- resp. sensitivitätsvermindernden Verfahren eingeklebt noch mittels solcher Verfahren eingegossen, sondern möglichst in ihrer "nativen" Form, d.h. in der Form, in der diese aus dem Herstellungsprozess hervorgeht, in ein System übertragen. Die Dichtung erfolgt dann alleine über die Elastizität der Materialien, insbesondere der Schichtstruktur. In allen hier beschriebenen erfindungsgemässen Systemen ist dies möglich, indem die Schichtstruktur nur mit dem Sensormodul in Verbindung gebracht (z.B. durch Beschichtung der Ableitvorrichtung/des Ableitelementes) oder ins Modul eingelegt, aber nicht weiter verändert oder bearbeitet wird. Dadurch wird grösstmögliche Selektivität resp. Sensitivität erreicht.

Die *selektive Schichtstruktur* kann auf einem geeigneten Träger z.B. auf einem planaren Wellenleiter, auf einer optischen Faser, auf einer reflektierenden Schicht, auf einer Diffusionsbarriere aufgetragen oder in einen Träger eingelegt vorliegen und auch als Schichtstruktur angeboten und in ein Modul eingesetzt werden. Solche Schichten werden in Fachkreisen allgemein als "Disposable layers" oder "disposable waveguides with target-analyte selective layers ") bezeichnet. Wenn ein Sensorelement verbraucht ist, können entweder nur das selektive Element, die Schichtstruktur, aber auch Teile des Moduls oder das ganze Modul ersetzt werden. Das einzelne Modul oder ein Set ist vorzugsweise so ausgelegt, dass es thermostatisiert werden kann, so dass es bei Einbau rasch messbereit ist.

Mindestens eine Komponente der Schichtstruktur bewirkt, dass eine Zielsubstanz oder eine Substanzklasse gegenüber von Begleitsubstanzen bevorzugt vom Sensor erkannt wird und zur Bildung einer Messgrösse führt. Die selektive Erfassung kann durch ein Verteilungsgleichgewicht der Substanz zu Gunsten der Schichtstruktur oder durch eine definierte chemische Wechselwirkung, Komplexbildung, reversible chemische Reaktion, zwischen der Zielsubstanz und einer Erkennungskomponente oder durch eine typische physikalische bzw. spektrale Eigenschaft induziert sein.

Als *Erkennungskomponente* kommen alle natürlichen und synthetischen sowie hybriden Moleküle in Frage. Solche Erkennungskomponenten sind beispielsweise Enzyme, Rezeptoren, Antikörper und deren Hybride oder Fragmente; Kohlenhydrate, Lektine, Lipide, Peptide, Proteine und deren Hybride oder Fragmente; synthetische Liganden, Bildner von Wasserstoffbrücken, Einschlussverbindungen, Solubilisate, Mizellen, Umkehrmizellen, Liposomen, Assoziate, Komplexe, metallorganische Komplexe, Reaktionsprodukte; Carrier, Ionophore, Reaktanden und chromophore Reaktanden, Chromoionophore, Redoxfarbstoffe, Elektronendonoren und -akzeptoren, "Charge-transfer"-Verbindungen, pH-Indikatoren, Komponenten zum Energie- und Ladungstransfer; Makromoleküle, Organe, Organellen, Mikroorganismen.

Der *Erkennungsschritt* kann durch Katalysatoren und weitere Hilfsstoffe (Weichmacher als Lösungsmittel, Polymere und Ionen) erleichtert resp. bevorzugt werden.

Die *Zielsubstanz* oder *Substanzklasse* umfasst infolge der Kombination verschiedener Detektionssysteme ein sehr breites Stoffspektrum. Als Beispiele seien hier genannt: Verwendung des Systems nach einem der Ansprüche 1 bis 5 zur Bestimmung anorganischer und organischer Ionen, geladener, ungeladener oder neutraler Moleküle, Salze, Isotope, Radikale, Zellen oder Zellbestandteile, Organismen, Mikroorganismen, Viren, Organellen, oder Rezeptoren im Untersuchungsmaterial.

Der mindestens eine Transduktionsschritt führt zur Bildung einer *Messgrösse* als Folge eines physikalisch chemischen Geschehens oder über die Ankopplung eines zweiten chemischen Transduktionsprozesses. Der *Transduktionsschritt* kann auch mehr als eines der Prinzipien in sich vereinen und kombinieren.

Als *Transduktionsschritt* wird z.B. die Bildung eines Grenzflächenpotentials in der Folge des Erkennungsschrittes/Verteilungsgleichgewichtes bezeichnet. Weitere Beispiele sind: die Generierung eines elektrischen Stromes als Folge eines Redoxprozesses, spektrale oder Frequenz-Aenderungen, die Aenderungen des Widerstandes bzw. der Leitfähigkeit, der Absorptions- oder Lumineszenzintensität, der Polarität, der optischen Dehnung, der Kapazität, oder die Aenderung der Masse, der Teilchenzahlen bzw. Quantenausbeuten als Folge des Erkennungsschrittes usw. Die *Messgrösse* besteht in einem Zählresultat, in der Quantifizierung der Absorptions-, Absorbanz-, Emissions- oder Frequenzänderung, in der Aenderung der Stromstärke *(I),* des Potentials *(EMK)* oder der Leitfähigkeit (σ), in der Aenderung der Masse (*m*), der Temperatur (*T*), der Abklingzeit (*t*) oder der Lebensdauer (*t*ₗᵢₘ) usw. Diese Messgrössen werden gegebenenfalls durch die Kalibration und den Vergleich mit einer Referenz in z.B. Konzentrationseinheiten bzw. in die geeignete Sprache der Information umgesetzt.

In der Folge werden anhand der Figuren und von Beispielen einige Sensoren resp. Module beschrieben um die Erfindung näher zu beschreiben. Diese Figuren und Beispiele sollen aber den Gegenstand der Erfindung in keiner Weise einschränken.

### Mehrdimensionales modulares Messystem

Figur 1 zeigt die Anordnung eines Messystems (Durchfluss) mit vier Modulen I, II, III und 13, einem potentiometrischen I und einem amperometrischen II Sensormodul je als Halbzellen ausgebildet, ein Modul mit dem Referenzelement 13 (zweite Halbzelle), sowie einem optischen Modul III. Die Module sind mit der Ableitung 12 quer zu einem Träger 1 angeordnet. Sie werden durch parallel zum Träger 1 angeordnete Schrauben 5, 6 dicht fixiert. Der Durchflusskanal 9 für die Probe/das Specimen eines einzelnen Moduls trifft exakt auf den des Nachbarmoduls und wird durch einen Gummiring gegen das Ausfliessen abgedichtet.

### Das potentiometrische Modul

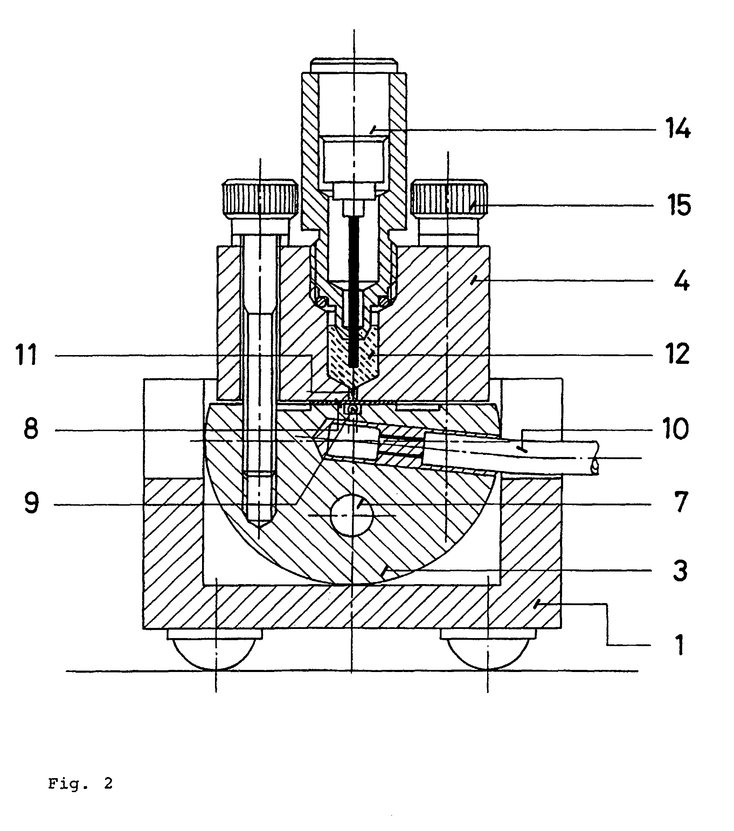

Die Ionen-selektive Schicht wird beim potentiometrischen Modul I auf ein Sensorfeld gelegt und steht über den an dieser Stelle offenen Probekanal 9 mit der Probe/dem Specimen in Kontakt (siehe Figuren 1 und 2). Das Modul wird in diesem Fall mit Wasser 7 thermostatisiert; es kann aber auch ein Peltierelement eingebaut oder das ganze System, sofern gewünscht, mit Luft thermostatisiert werden. Basisteil 3 und Kopfteil 4 können beispielsweise unabhängig voneinander produziert und nach dem Einbringen der Schichtstruktur 8 mittels Befestigungselement, z.B. Schrauben 15, zusammengefügt / verschlossen werden. Das Kopfteil 4 enthält das Sensorelement mit der Schichtstruktur 8 und den Körper der Ableitvorrichtung 14, resp. die Ableitvorrichtung/das Ableitelement 12, hier ein Ableitelektrolyt und eine Ableitelektrode.

Dieses Kopfteil 4 ist vorzugsweise so gestaltet, dass nur die Bestückung durch Sensorelemente nicht jedoch die Kunststoffkonstruktion wesentlich ändern. So können Kopf- und Basisteil in hohen Stückzahlen und damit kostengünstig produziert werden. Ferner wird dadurch die Austauschbarkeit der Module und damit die Flexibilität des Systems gewährleistet.

### Das Amperometrische Modul (Mediated biosensor electrodes)

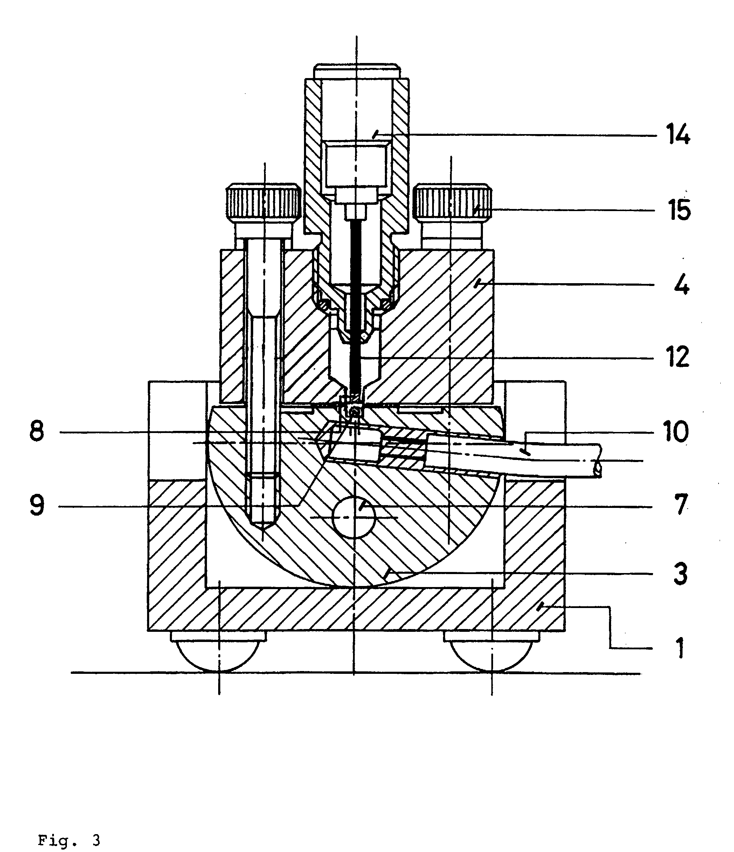

Es hat sich nun gezeigt, dass ein amperometrischer Biosensor Modultyp nach demselben Bauplan wie das potentiometrische Modul konstruiert werden kann (vgl. Figuren 1 und 3). Die Ionen-selektive Schichtstruktur 8 des potentiometrischen (ISE)-Moduls wird dabei durch die Schichtstruktur 8 des Biosensors ersetzt. Das Ableitelement 12 besteht hier vorzugsweise aus einem Platindraht, der in direktem Kontakt mit der Schichtstruktur resp. der "Paste" steht. Die "Paste" enthält das gewünschte Enzym als Erkennungskomponente, sowie vorzugsweise TTF/TCNQ (Tetrathiafulvalen-p-tetracyanochinondimethan) als Mediatoren und ein Bulkmedium, z.B. Silikonoel. Entsprechende Pasten wurden bereits beschrieben [vgl. Korell, U.; Spichiger, U.E. Electroanalysis 6 (1994) 305-315]. Die Paste kann z.B. in Form einer Tablette angepasster Viskosität oder zusammen mit einem Träger (z.B. aus Polyolefin) ins Kopfteil 4 eingesetzt, aufs Sensorfeld aufgelegt oder mit der auswechselbaren Ableitelektrode 12 verbunden eingebracht werden. In einer bevorzugten Ausführung bildet der Probekanal 9 zonenweise die Gegenelektrode. Beispielsweise besteht er zonenweise aus einem Platinröhrchen.

Das Referenzmodul 13 bleibt sich gleich wie beim potentiometrischen Modultyp. Es besteht vorzugsweise aus einer "free-flow free-diffusion" Elektrode, wie sie (nichtmodular) in Dohner, R.; Wegmann, D.; Morf, W.E.; Simon, W. Anal. Chem. 58 (1986) 2585-2589 beschrieben ist. Diese Elektrode hat sich im kontinuierlichen Betrieb als äusserst robust und interferenzarm erwiesen.

### Das optische Sensormodul

Es wurde ebenfalls gefunden, dass auch optische Sensoren für die kontinuierliche, robuste Messtechnik verwendet werden können. Der Aufbau eines erfindungsgemässen Moduls ist wie folgt:

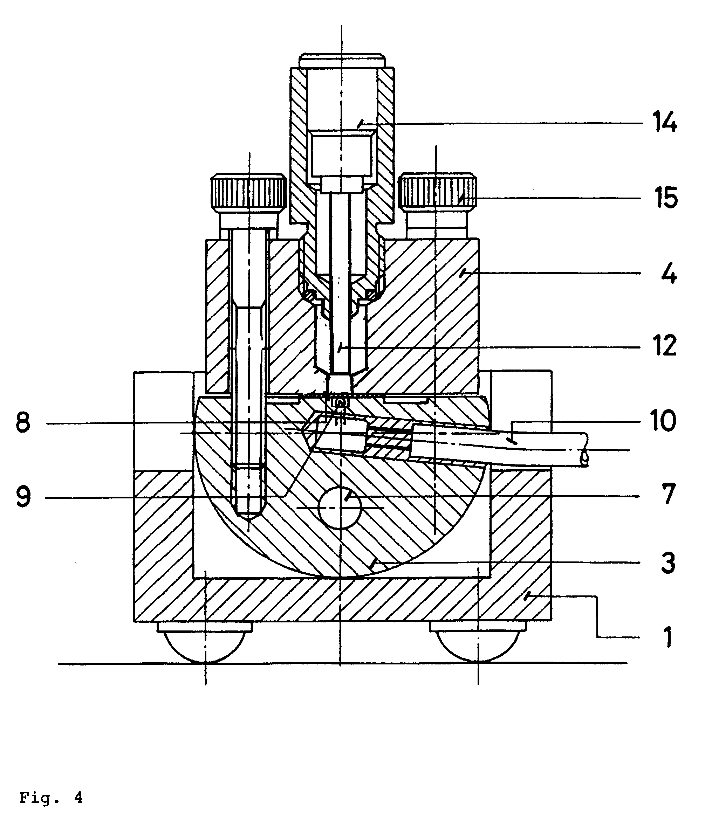

Das optische Sensormodul ist wiederum so konstruiert, dass lediglich die Sensorelemente (Schichtstruktur 8 und Ableitvorrichtung 12) im Kopfteil des Moduls ändern (vgl. Figuren 1, 4 und 5). Vorzugsweise soll ein wegwerfbares (disposable) Element als Wellenleiter verwendet werden, auf dem die Schichtstruktur z.B. aufgezogen bzw. mindestens ein Selektivitätsprinzip realisiert/angekoppelt wird. Der Wellenleiter kann gleichzeitig den Transduktionsschritt ermöglichen und als Ableitvorrichtung 12 für die Ableitung zuständig sein. Wellenleiter können z.B. beschichtete Gitterkoppler sein, welche auf das Sensorfeld, die selektive Schichtstruktur 8, aufgebracht oder im Kopfteil 4 eingelegt/eingebaut werden. Die Messgrösse bei optischen Sensoren kann z.B. durch Messungen der Absorpiton/Absorbanz im ATR-Modus (attenuated reflection) [vgl. Spichiger, U.E.; Freiner, D.; Lerchi, M.; Bakker, E.; Dohner, R.; Simon, W. SPIE Vol 1796 (1992) 371-382], durch Messungen von Brechungsindexänderungen [vgl. Freiner, D.; Kunz, R.E.; Citterio, D.; Spichiger, U.E.; Gale, M.T. Sensors and Actuators B 29 (1995) 277-285], durch Messung der optischen Dehnung, der Phasenverschiebung, der Reflektion oder der Lumineszenz-Zerfallszeit oder durch die Ableitung der Lumineszenzemission gebildet werden.

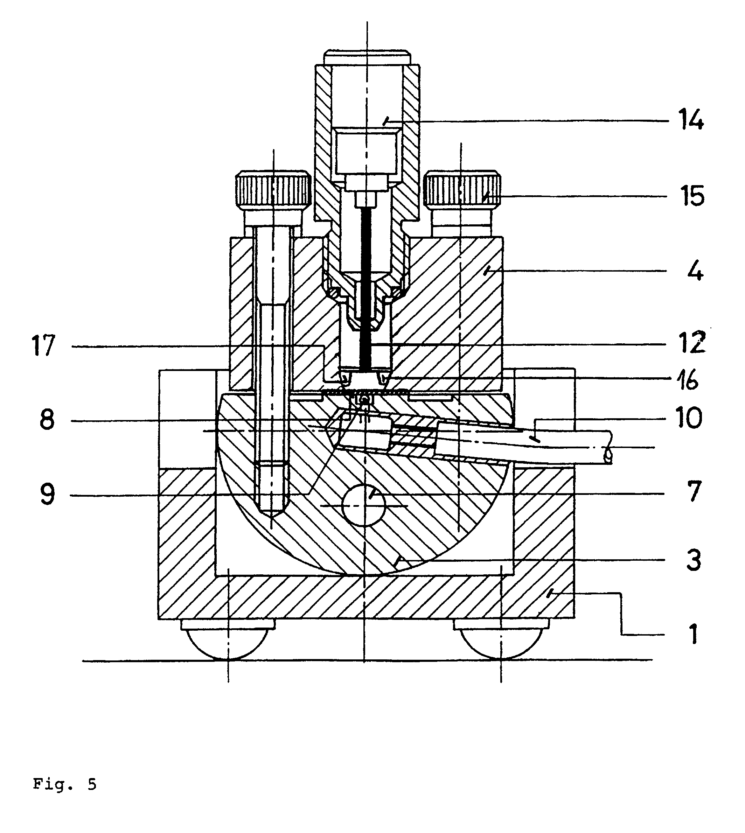

Die Lichtenergie kann von ausserhalb des Moduls über eine optische Faser oder andere optische Wellenleiter eingestrahlt werden oder die Lichtquelle kann durch die Implementierung von Dioden (Emissionsdiode 17 , LED) in den Kopf des Moduls integriert werden. Auch eine Detektordiode 16 kann in den Kopf des Moduls integriert werden (vgl. Figur 5). Die Ableitvorrichtung 12 ist beispielsweise ein elektrisches Kabel.

In allen diesen Modultypen können die Schichtstruktur 8 und die Ableitvorrichtung 12 getrennt, d.h. einzeln auswechselbar vorliegen. Dadurch wird die vielfältige Verwendbarkeit des Systems optimiert und die Kosten minimiert. Durch den ähnlichen Aufbau der Module lassen sich diese optimal kombinieren, so dass sie die simultane und kontinuierliche Messung verschiedener Parameter in der Probe ermöglichen.

Es ist ferner möglich, das Ableitelement 12 auswechselbar zum Körper 14 zu konstruieren, so dass dieses Element ausgetauscht werden kann, ohne einen gegebenenfalls teure Elektronik enthaltenden Kopf 14 ebenfalls auswechseln zu müssen.

### Beispiele

### Erkennungskomponenten

Wesentlich für jedes Modul ist das Selektivitätsprinzip bzw. der Erkennungsschritt, beispielsweise durch eine Erkennungskomponente.

### Potentiometrie:

Speziell für die Potentiometrie geeignete Erkennungskomponenten sind selektive Komplexbildner. Einige werden in der Folge näher beschrieben.

Die *Magnesium-selektiven Komplexbildner ETH 3832 und ETH 5506* haben sich als die bei weitem effektivsten Liganden erwiesen [vgl. Spichiger, U.E.; Rumpf, G.; Haase, E.; Simon, W.; Schweiz.Med.Wschr. 121 (1991) 1875-1879; Spichiger U.E.-, Electroanalysis 5 (1993) 739-745]. Allerdings ist deren optimale Selektivität gebunden an eine optimierte Zusammensetzung der Schicht oder Membran [vgl. Spichiger U.E.; Eugster, R.; Citterio, D.; Li, H.; Schmid, A. und Simon, W. (1993), Magnesium activity measurements: facts and enthusiasm, In: Golf, S.; Dralle, D.; Vecchiet, L. (Ed.), *Magnesium 1993,* John Libbey & Co Ltd., Seiten 49-60]. Die zur Zeit optimale Zusammensetzung ist die folgende:
Ligand ETH 3832 oder ETH^{T} 5506 1 - 2 Gew.-%
Weichmacher ETH 8045 ca. 62 Gew.-%
KTpClPB (Kalium tetrakis[3,5-bis(trifluoromethyl)phenyl]borat) 155 mol % relativ zum Ligand
PVC bzw. Hydroxy-PVC ca. 35 Gew.-%.

Die Liganden sind beschrieben in Spichiger U.E.; Eugster, R.; Citterio, D.; Li, H.; Schmid, A. und Simon, W. (1993), Magnesium activity measurements: facts and enthusiasm, In: Golf, S.; Dralle, D.; Vecchiet, L. (Ed.), *Magnesium 1993,* John Libbey & Co Ltd., Seiten 49-60 und O'Donell, J.; Hongbin,L.; Rusterholz, B.; Pedrazza, U.; Simon, W.; Anal. Chim. Acta 281 (1993) 129-134, die Weichmacher unter Eugster, R.; Rosatzin, T.; Rusterholz, B.; Aebersold, B.; Pedrazza, U.; Rüegg, D.; Schmid, A.; Spichiger, U.E.; Simon, W.; Plasticizers for liquid polymeric membranes of ion-selective chemical sensors, *Anal. Chim.* Acta, 289(1994) 1-13. Anstelle von ETH 3832 können auch weitere lipophile, höher alkylierte Derivate verwendet werden wie z.B. das Dodecyl-Derivat ETH 5405 (Spichiger U.E., *Electroanalysis,* 5 (1993) 739-745). Anstelle von ETH 8045 als Weichmacher kann ETH 5373, ETH 220 u.a. eingesetzt werden.

Zur Herstellung der selektiven Schicht werden die Komponenten in einem flüchtigen organischen Lösungmittel gelöst (z.B. THF, Chloroform, Methylenchlorid, Cyclohexanon und Gemische). Die selektive Schicht wird gemäss Oesch, U.; Brzozka, Z.; Xu, A.; Rusterholz, B.; Suter, G.; Pharm, H.V., Welti, D.H.; Ammann, D.; Pretsch, E.; Simon, W.; Anal.Chem. 58 (1986) 2285 hergestellt. Die Schicht/Membran kann zur Erhöhung der Biokompatibilität der Oberfläche z.B. mit einem Polyurethan-Präparat, in THF gelöst, überzogen werden. Dazu wird die Membran auf einem Glasplättchen trocknen gelassen. Das Glasplättchen, z.B. mit einem Durchmesser 1.8 cm, wird auf einer mit 600-1000 rpm rotierenden Scheibe fixiert. Nun wird mit einer Spritze 0.5-1.0 ml einer Lösung mit 0.3-3.0 Gew.-% Polyurethan (z.B.Tekoflex EG-80A, EG-85A (Thermedics, Woburn)) aufgespritzt und danach eintrocknen gelassen. Derart wird eine *Schichtstruktur* mit vorteilhaften Eigenschaften erzeugt. Der Vorteil dieser Behandlung ist, dass eine gegenüber Adsorptionen aus der biologischen Probe robuste Oberfläche geschaffen wird, ohne die Eigenschaften der selektiven Schicht oder deren Ansprechzeit wesentlich zu verändern.

Jede Veränderung der Zusammensetzung kann zum teilweisen Verlust der Selektivität und Sensitivität führen. Eine Synthese für den Liganden ETH^{T} 5506 ist in O'Donnell, J.; Hongbin, L.; Rusterholz, B.; Pedrazza, U.; Simon, W.. Anal. Chim. Acta 281 (1993) 129-134 beschrieben. Diese schliesst zahlreiche Zwischenprodukte ein, welche chromatographisch zu reinigen sind. Die *Synthese* wurde deshalb in den folgenden Schritten vereinfacht:

Entschützung des mit einer Phthaloyl-Schutzgruppe geschützten 1,3,5-tris(aminopentyl)benzols durch Reduktion des Phthalids zum Triamidtriol (NaBH4, *i*-PrOH/H₂O 17/3; 12h RT) mit 92% Ausbeute und Pyrolyse des Triamidtriols (170-200°C, 4 atm im Kugelrohr) mit Abdestillation des Phthalids und anschliessender Destillation des Triamins (200°C, Hochvakuum im Kugelrohr) im selben Schritt mit 95% Ausbeute.

Auch die Synthese des als 4-Nitrophenylester aktivierten 1-Adamantyl-methyl-malonmonoamid-Bausteins wurde effizienter gestaltet. Die Synthese des Adamantylamins aus *N*-Adamantylmethyltrifluoroacetamid liefert schlechte und nicht reproduzierbare Ausbeuten des sek. Amins. Diese Probleme konnten gelöst werden, indem die Hydrolyse des Trifluoroacetamids mit KOH/18-crown-6 in THF ausgeführt wurde. Als Ausgangsprodukt wurde das wesentlich billigere Adamantylhydrochlorid verwendet und dieses mit Methyliodid methyliert. Das methylierte Adamantylamin wurde mit dem Malondimethylester am Rückfluss kontinuierlich zum Adamantyl-methyl-malondiamid umgesetzt. Das Produkt fällt aus der Mutterlauge aus und kann abfiltriert werden (80% Ausbeute). Unreagiertes Adamantyl-methylamin kann leicht rückisoliert werden. Der als 4-Nitrophenylester aktivierte Baustein 1-Adamantylmethyl-malonmonoamid wird mit 40% Ausbeute über beide Stufen erhalten.

Liganden für die Bestimmung von *Schwermetal*len insbesondere *Blei:*

Zur Bestimmung von Blei in Trink-, Grund- und Abwasser wurden lipophile 19-Di- und trithiacrown-6 Isologe als PbOH⁺-selektive Liganden entwickelt. Früher entwickelte Liganden zeigten eine Kupferselektivität, welche für die Analyse von Wasserproben ungenügend war und die selektive Analyse von Bleiionen nicht erlaubten [vgl. Lerchi, M.; Bakker, E.; Rusterholz, B.; Simon, W. Anal. Chem. 64 (1992) 1534 ff.; KTI-Projekt Nr. 2692.1; EUREKA-Projekt "ASREM" EU 1013]. Die Liganden 18,18-Bis-(dodecyloxymethyl)-1,4,13,16-tetraoxa-7,10-dithiacyclononadecan (5) und 18,18-Bis-(dodecyloxymethyl)-1,7,10,16-tetraoxa-4,13-dithiacyclononadecan (7) wurden synthetisiert und in klassische Ionen-selektive PVC-Membranen mit Ethyl-hexylsebacat (DOS) bzw. Dibutylsebacat (DBS) als Weichmacher und 30 mol% Kalium tetrakis[3,5-bis(trifluoromethyl)-phenyl]borat eingebracht. Die Selektivitätsmuster der beiden Liganden wurden mit der Methode der getrennten Lösungen nach IUPAC potentiometrisch bestimmt. Sie zeichnen sich durch eine Diskriminierung von Kupferionen um einen Faktor von 10^{-5.8} aus und durch eine Diskriminierung von Alkaliionen um einen Faktor von < 10⁻³ (molale Konzentrationseinheiten). Auch hier kann der bedeutende Einfluss des Weichmachers bzw. des Mediums, der Umgebung, beobachtet werden. Bevorzugte Weichmacher resp. Medien sind ETH 8045, ETH 220 [beschrieben in Eugster, R.; Rosatzin, T.; a.a.O. S. 26, Zeile 27], Bis(2-ethylhexyl)adipate (Fluka Chemie AG, Selectophore).

### Synthese und Verwendung von Chromogenen Liganden für Karbonat, Feuchtigkeit und primäre Alkohole

Verschiedene Trifluoroacetylanilin Derivate wurden von Keller-Lehmann,B.; Diss ETH Nr. 9255, *Trifluoroacetophenon-Derivate als carbonatselektive Ionophore in PVC*-Flüssigmembranelektroden (1990) hergestellt und als reaktive Erkennungskomponenten für die selektive Bestimmung von Karbonat, Feuchtigkeit und primäre Alkohole vorgeschlagen. Die Erkennungsreaktion besteht in der Bildung eines Halbazetals und ist reversibel. Die Liganden wurden in Optoden- und Elektrodenmembranen mit Erfolg eingesetzt [vgl. Wild, R.; Citterio, D.; Spichiger, J.; Spichiger U.E. J. Biotech. 50 (1996) 37-46]. Für die Detektion in optischen Sensoren wird bei einer Wellenlänge von ca. 305 nm die Abnahme der Absorbanz der aromatischen Trifluoroacetylgruppe als Folge der Halbazetalbildung beobachtet. Diese Wellenlänge eignet sich jedoch schlecht für die Konstruktion von mobilen, kostengünstigen Sensorelementen. Es wurde daher eine Gruppe von *Chromogenen Reaktanden* entwickelt, welche im sichtbaren Bereich des Spektrums absorbieren und dennoch die Selektivität gegenüber den erwähnten Substanzen beibehalten. Es sind dies N,N-disubstituierte-aminophenyl-4'-trifluoracetylazobenzole, N,N-disubstituierte-aminonaphthalin-4'trifluoracetyl-azobenzole, 4-Trifluoroacetyl-4'-(N,N-disubstituierte-amino)stilbene und disubstiuierte -pamino-trifluoroacetylstilbene, wie *N,N*-dioctylaminophenyl-4'-trifluoroacetyl-azobenzen, *N,N*-dioctylaminonaphtalin-4'-trifluoroacetyl-azobenzen, 4-Trifluoroacetyl-4'-(di-*N*-octylamino)stilben und p-Dialkylamino-trifluoroacetylstilbene. Diese werden hergestellt nach den Schemata 1 bis 3. Die Synthese der Stilbenderivate erfolgte aus 1-Iodo-4-dialkylamino-benzol und 1-Brom-4-ethenylbenzol mit nachfolgender Azetylierung mit Trifluoroacetylmethylester. Die Substitute sind langkettig und lipophil. Sie unterscheiden sich in der elektroneninduzierenden Wirkung. Ueblicherweise sollte ihre Lipophilie mind. 3 nach Hansch sein.

Die chromophoren Reaktanden können kovalent z.B. an Acrylat- und Methacrylat gebunden und in Polymermembranen immobilisiert werden.

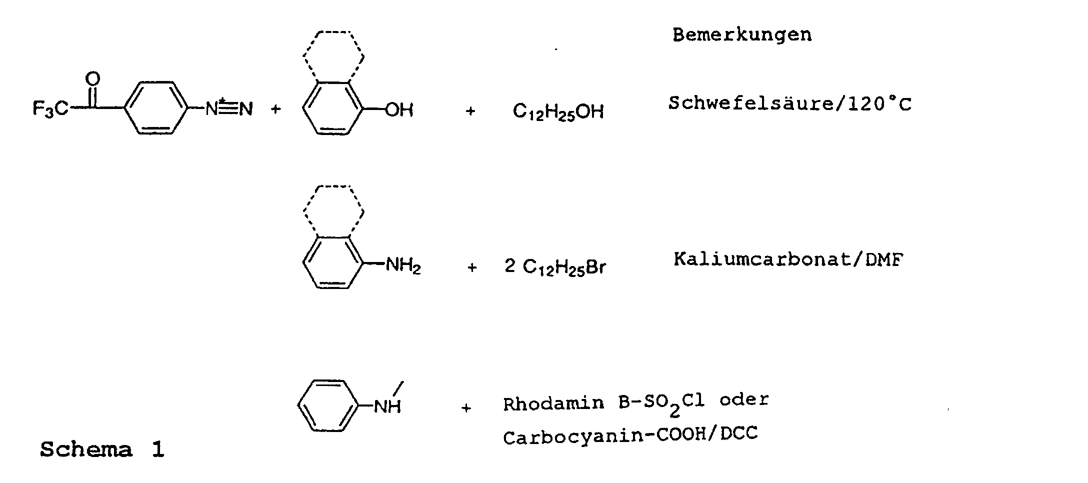

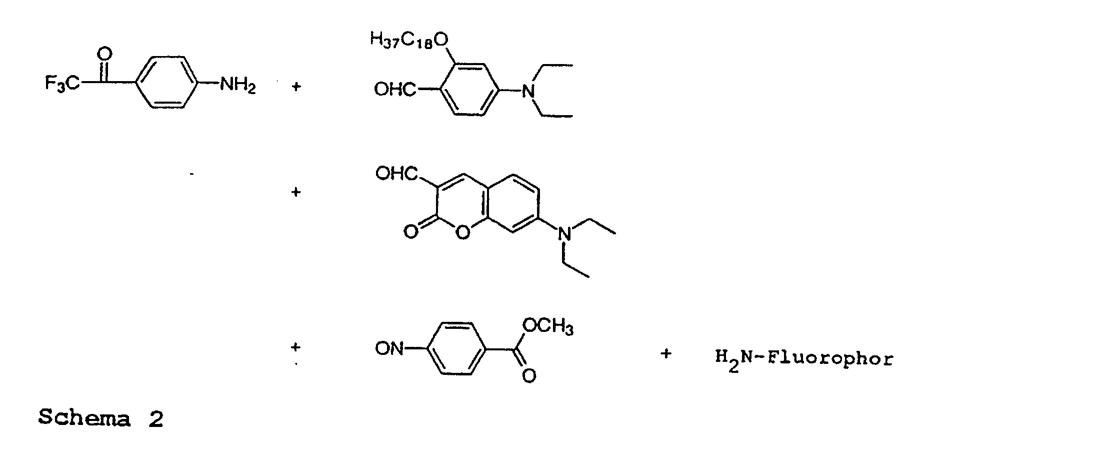

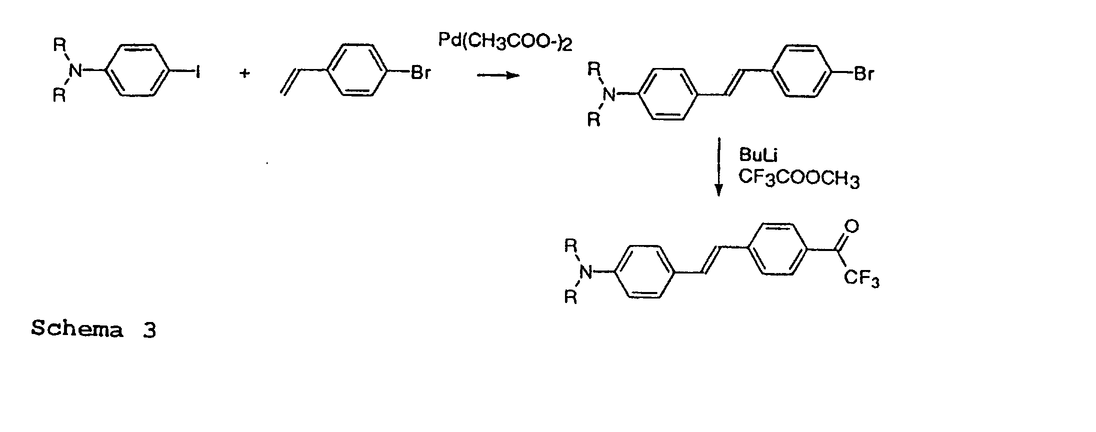

### Herstellung von optischen selektiven Schichten für die Nitrit-, NOₓ- und Chlorid-Bestimmung

Auf der Basis von bekannten Nitrit-, NOₓ- und Chlorid-selektiven Liganden (Nitritionophore I, Fluka Chemie AG, Buchs, Schweiz; Chlorid-selektiver Ligand ETH 9033 [vgl. Rothmaier, M.; Quecksilberorganische Verbindungen als neutrale Anionocarrier in ionenselektiven PVC-Flüssigmembranen. Diss. ETH Nr. 10812, 1994] wurden Schichten/Membranen für optische Sensorelemente hergestellt. Dazu wurde eine H⁺-selektiver Chromoionophor (Chromoionophor I, II und VI für Nitrit; Chromoionophor III für Chlorid, Fluka Chemie AG, Buchs, Schweiz) mit dem Nitrit- bzw. Chlorid-selektiven Liganden und, wo dies zur Ladungskompensation notwendig ist, gemeinsam mit anionischen bzw. kationischen lipophilen Hilfsstoffen in einer DOS-plastifizierten PVC-Schicht gelöst. Die Absorbanzänderung als Folge der Koextraktion des Anions zusammen mit H⁺ aus der gepufferten Probelösung wird photometrisch bestimmt und folgt der Konzentrationsänderung des Anions.

Für *Nitrit* liegt der Messbereich zwischen 0.24 bis 5000 mg kg⁻¹, Chlorid wird mit einem Selektivitätsfaktor von 10^{-2.9} (molale Einheiten) diskriminiert. Der Messbereich und, vom Messbereich abhängig, auch die Ansprechgeschwindigkeit ändern mit dem verwendeten Chromoionophoren; die Chromoionophoren zeigen verschiedene Stabilität. Die Implementierung mehrerer Optodenschichten für die Nitritbestimmung oder einer Nitrit-selektiven Optode und Elektrode in einem System kann, der Fragestellung entsprechend, wünschenswert sein. Die Nitritselektive Membran mit Chromoionophore VI als Indikator zeigt eine Lumineszenzemission im sichtbaren Bereich des Spektrums und kann deshalb auch als Lumineszenz-aktive Schicht eingesetzt werden.

Die oben erwähnten Liganden haben bevorzugte Anwendungsgebiete, sie können aber generell in optischen, potentiometrischen und beliebigen weiteren Systemen als Selektivitätsprinzip zur Anwendung kommen, gegebenenfalls in Kombination mit weiteren Schichten, gegebenenfalls auch zur Bestimmung von Gasen und flüchtigen Substanzen.

### Sauerstoffbestimmung mit optischen Sensoren

Insbesonders für die Sauerstoffbestimmung mit optischen Sensoren wurde ein neues Selektionssystem entwickelt.

Ru(II)-Komplexe mit organische Liganden, insbesondere Ru(II)-(4,7-diphenyl-1,10-phenantrolin)₃ Komplexe wurden in den letzten Jahren für die Bestimmung des Sauerstoff-Partialdruckes in Lösung eingeführt. Der Ru(II)-Komplex dient dabei gleichzeitig als *Erkennungs- und Transduktionskomponente.* Dabei wird ausgenützt, dass die Lumineszenzemission der Komplexe durch O₂ gequencht d.h. unterdrückt wird. Die Anregung findet bei 470 nm statt, das Emissionsmaximum liegt bei 607-624 nm je nach Umgebung/Bulk der Schicht.

Die bekannten Ru(II)-Komplexe sind trotz ihrer Assoziation an organische Liganden sehr hydrophil und können deshalb schlecht in einer apolaren Schicht gelöst werden. Erst durch Einführung lipophiler Gruppen am Phenylrest, beispielsweise die Alkylierung des Phenylrestes in 4'-Position wurde dies möglich. Insbesondere Propyl- bis Dodecyl-, speziell bevorzugt Propyl- bis Octyl-, ganz speziell bevorzugt Propyl- bis Heptyl-Derivate sind geeignete Indikatoren für gelösten Sauerstoff in apolarer Membranumgebung. Das Auswaschen kann durch Ionenpaarbildung mit lipophilen Anionen bzw. Immobilisierung weiter verlangsamt werden. Solche Komplexe lassen sich in 3 Schritten gemäss Schema 4 und nachfolgendem Reinigungsprozess herstellen. Die Herstellung von 4-Heptyl-β-chlorpropiophenon [nach Case, F., Strohm, P.F., J. Org. Chem. 27 (1962) 1641 ff.] erfolgte aus 1-Phenylheptan und 3-Chlorpropionsäurechlorid, die Herstellung von 4,7-bis(4'-heptylphenyl)-1,10-phenanthrolin [nach Lund, G.K., Holt, S.L., J. Chem. Eng. Data, 26 (1981) 277 ff.] aus 1,2-Phenylendiamin und 4-Heptyl-β-chlorpropiophenon, und die Herstellung des Ruthenium(II)-Komplexes, Ru(II)[-4,7-bis(4'-heptylphenyl)-1,10-phenanthrolin]₃ [nach Wolfbeis, O.S., et al., Anal. Chem. 60 (1988) 2028 ff.] aus 4,7-bis(4'-heptylphenyl)-1,10-phenanthrolin und RuCl₃.

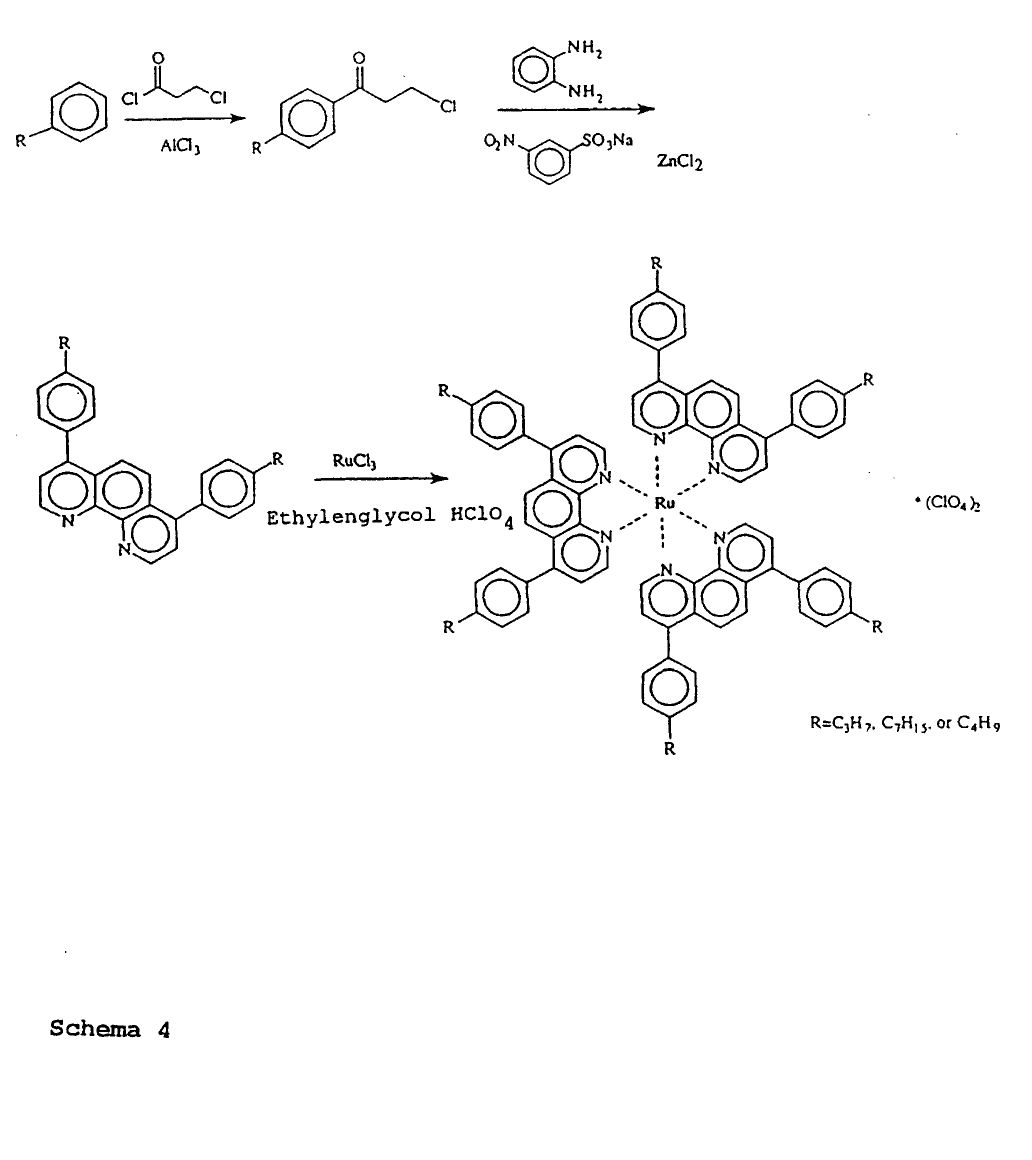

Die Reinigung erfolgte mit Hexan/Wasser, Aceton/Wasser und Aktivkohle, Umkristallisieren aus Aceton/Wasser, Säulenchromatographie mit Dichlormethan/Aceton oder Chloroform.

Analoge Synthese für das Propyl- oder Butyl-Derivat wird ausgehend von 4-Propyl-β-chlorpropiophenon resp. 4-Butyl-β-chlorpropiophenon durchgeführt.

Die Anregung in erfindungsgemässen Modulen wird vorzugsweise über einen Wellenleiter oder eine Diode im Kopfteil des Sensormoduls vorgenommen. Die Luminszenzemission wird auf dieselbe Art abgeleitet. Die Messgrösse bzw. das Messignal wird durch die Aenderung der Emissionsintensität bei 607 bis 624 nm gebildet. Diese Messgrösse wird mit dem Signal einer Lösung mit bekanntem O₂-Partialdruck verglichen was zur Information über die Zielsubstanz führt.

Weiter wurde auch die Lebensdauer der Lumineszenzemission in plastifizierten Polymeren untersucht. Diese zeigte gute Resultate. Die Messung der Abklingzeit eignet sich speziell zur Realisierung von *kalibrationsfreien* Systemen. Komplexe der genannten Liganden mit Lanthaniden (z.B. Eu, La) führen zu weiteren attraktiven Verbindungen mit verlängerter Lebensdauer für die Messung der Lumineszenz-Abklingzeit.

Der Einsatz der Ru(II)-Komplexe in der organischen Phase von Umkehrmizell-Systemen [vgl. Vaillo et al, AMI 2/3 (1995) 145-153] führt zu einfachen und direkten Indikatorschichten für die Bestimmung der Aktivität von Oxidasen (z.B. für Laktat, Glutamat, Glucoseoxidase, Hypoxanthin, Peroxidase) z.B. über den Sauerstoffverbrauch.

Im Gegensatz zu älteren Selektivitätsprinzipien, welche aus 2 Schichten bestehen, die nach den vorne beschriebenen, nachteiligen Stand der Technik-Verfahren hergestellt wurden, ist das Einschichtsystem effizienter und daher robuster gegenüber variablem O₂-Druck.

### Amperometrische Biosensoren

Bestandteil der selektiven Schichtstruktur eines amperometrischen Biosensors ist z.B. eine Paste, welche einen organischen Mediator als Elektronentransfer-Katalysator enthält und gemäss Korell, U.; Spichiger, U.E. Electroanalysis 6 (1994) 305-315; Korell, U.; Spichiger, U.E. Anal. Chem. 66 (1994) 510-515 herstellbar ist. Das Redox-aktive Enzym wird in die "Paste" eingebracht und zeigt in dieser Umgebung eine optimale Lebensdauer. Der Bulk der "Paste" dient dabei als Reservoir aus welchem aktives Enzym nachgeliefert werden kann. Die "Paste" kann in die dafür vorgesehen Aussparung ins Kopfteil des Sensormoduls eingebracht werden (siehe Figuren 1 und 3). Die Oberfläche bildet das Sensorfeld wo Probe und selektive Schichtstruktur miteinander in Kontakt stehen. Vorzugsweise ist die Paste hochviskos bis elastisch, so dass sie direkt als Tablette vorliegt, oder sie wird mittels eines Trägers in auswechselbare Tablettenform gebracht.

### Glucose-Bestimmung

Eine Paste, welche 20 Gew.-% Glucose-Oxidase (GOD EC 1.1.3.4) in Siliconoel/TTF/TCNQ enthält konnte über 2.5 Monate kontinuierlich betrieben werden (Standard-Parameter: T 25°C; Arbeitsspannung U = +200 bis -200 mV; Puffer: 50 mM Imidazol/ 50 mM NaCl, pH 7.0; Flussrate (Förderrate der Peristaltikpumpe: 0.025ml/min). Der dynamische Messbereich eines solchen Moduls im erfindungsgemässen kontinuierlichen System erwies sich als gut geeignet im Konzentrationsbereich von ca. 1 bis 100 mM (U= - 100mV gegenüber SCE), mit der rotierenden Stabelektrode liegt die Detektionsgrenze bei 1 µM (200mV, 25Hz). Die Paste kann auch für die Massenproduktion auf einem Trägermateriel (vorzugsweise dem Elektrodenmaterial) aufgedruckt werden und stückweise als selektive Schichtstruktur für die Belegung des Sensorfeldes verwendet werden. Anstelle von Siliconoel sind weitere Bulkmaterialien (vergl. potentiometrische Ionen-selektive Membranen) verwendbar. Zur Erhöhung der Viskosität und mechanischen Robustheit wird ein Hilfstoff, z.B. ein Polymer wie PVC, ins Silikonoel eingebracht.

Anhand eines *Phosphat-selektiven Sensors* konnte gezeigt werden, dass auch die Herstellung einer bienzymatischen Schicht, in welcher zwei Enzyme in dieselbe Schicht eingebracht werden, möglich ist [vgl. Müller, J.P.; *Entwicklung eines phosphatsensitiven bienzymatischen-amperometrsichen Biosensors.* Diplomarbeit ETHZ, 1994]. Das Phosphat-selektive Sensorelement enthält 1 Teil TTF/TCNQ (Tetrathiafulvalen-p-tetracyanochinodimethan) hergestellt nach Ferraris et al. [J. Am. Chem. Soc. 95 (1973) 948] in 1.6 Teilen Siliconoel. Mit dieser fein zerriebenen Paste werden z.B. 3.18 Gew.-% Xanthin-Oxidase (EC 1.1.3.2) und 4.35 Gew.-% Nucleosid-Phosphorylase (EC 2.4.2.1.) entsprechend 7.53 Gew.-% Gesamtlyophilisat-Gehalt vermischt. Die Paste wird in die dafür vorgesehene Aussparung (Sensorfeld) eine modulare Stabelektrode eingebracht und diese in der Anordnung einer drehenden Scheibenelektrode geprüft. Die Paste wird bei -30°C gelagert. Vor der Kalibration und Prüfung der Elektrode wird diese in einer Pufferlösung pH 7.0, welche 50 mmol L⁻¹ Imidazol, 50 mmol L⁻¹ Natriumchlorid und 1.2 mmol L⁻¹ Inosin enthält, 16 h konditioniert. Für die Funktionsprüfung wird dieser Pufferlösung alle 2 Minuten ein Inkrement Phosphatlösung zugefügt, das zu einer Konzentrationserhöhung um 1.0 µmol L⁻¹ bzw. 5 µmol L⁻¹ Phosphat führt. Die Aenderung des Anoden-Stromes wird bei einer Rotationsfrequenz von 25.0 ± 0.05 Hz gemessen. Die Konzentrationsänderung konnte bei einer angelegten Spannung von -50.0±0.2 mV innert <15s gemessen werden.

Alle drei vorgestellten Sensorelemente können gleicherweise als stabförmige Modultypen konstruiert und z.B. in einer Sonde vereint in einen Reaktor eingebracht oder in-line mit dem Reaktor verbunden werden. Jedes System kann mehrere Module eines Typs in Kombination mit Modulen eines anderen Typs umfassen. Die mehreren Module des gleichen Typs können sich beispielsweise durch verschiedene Erkennungskomponenten unterscheiden. Die Verarbeitung der aus den einzelnen Modulen austretenden Signale resp. die Ausgabe des Messresultats erfolgt nach bekannten Verfahren. So kann beispielsweise über eine Ableitvorrichtung mindestens eine Messgrösse aus einem Messmodul oder eine Bezugsgrösse aus dem Referenzmodul auf eine Vorrichtung zur Verarbeitung der Messgrössen ableiten wird, derart, dass die Messgrösse zur Prozesssteuerung verwendet werden kann.

Die Information über die Substanz wird gewonnen, beispielsweise als Menge, Konzentration resp. Konzentrationsänderung, als Zählresultat, Zeit, Aktivität oder aktive Molalität.

Das System eignet sich zur Bestimmung anorganischer und organischer Ionen, geladener, ungeladener oder neutraler Moleküle, Salze, Isotope, Radikale, Zellen oder Zellbestandteile, Organismen, Mikroorganismen, Viren, Organellen, oder Rezeptoren und ist verwendbar in der medizinischen Diagnostik und Behandlung, in der Dialyse und Haemodialyse, in der klinischen Analytik, in der Umwelttechnik, Bautechnik, Reinraumtechnik, Abwasser-, Grund- und Trinkwasserkontrolle, in der Lebensmitteltechnologie und -produktion, in der Agrartechnologie, insbesonder in der Hors-sol-Kultur-Technik, in der Lebensmitteltechnologie, in der Biotechnologie und der chemischen Prozesstechnik, in der Pharmaforschung und -produktion, der Arzneimitteltechnologie und der Arzneimittelkontrolle, sowie in der Textiltechnik und in der Waschmittel- und Reinigungs- und Tensidtechnologie.

Die selektiven Schichtstrukturen können in diversen Dicken vorliegen. Durch die Auswechselbarkeit können sie somit leicht für spezielle Anwendungen optimiert werden. Die Abdichtung zwischen Schichtstruktur und Probekanal erfolgt üblicherweise lediglich durch Dichtungen, z.B. aus Kunststoff, und Anpressen der Schichtstruktur mittels des Kopfteils resp. Ableitelementes auf diese Dichtung. Die Verwendung von selektiven Schichtstrukturen führt dazu, dass ohne Reagentien gearbeitet werden kann. In vielen Fällen wird eine Verdünnung und Pufferung des Specimens nötig sein.

## Patentansprüche

1. Modulares System zur kontinuierlichen reagenzienfreien Bestimmung mindestens einer Substanz, **dadurch gekennzeichnet, dass** es mindestens zwei Messmodule (I, II, III) gleichen oder verschiedenen Modultyps zur simultanen Gewinnung von mindestens zwei Informationen im Durchfluss aufweist, wobei jedes Modul mindestens eine auswechselbare selektive Schichtstruktur (8) und eine Ableitvorrichtung (12) enthält, wobei die Schichtstruktur (8) einen selektiven Erkennungsschritt ermöglicht und eine kontinuierliche Messung zulässt, wobei das Modul einen Kanal (9) für den Durchfluss der Probe enthält, derart, dass das Innere des Probekanals (9) mit der selektiven Schichtstruktur (8) des jeweiligen Moduls in direktem Kontakt steht und wobei die Dichtung zur selektiven Schichtstruktur vor allem über die Elastizität der Schichtstruktur erfolgt.

2. Modulares System gemäss Anspruch 1, **dadurch gekennzeichnet, dass** es mehrdimensional ist, d.h. Messmodule (I, II, III) mindestens zweier Modultypen umfasst.

3. Modulares System gemäss Anspruch 1 oder 2, **dadurch gekennzeichnet, dass** die Messmodule ausgewählt sind aus der Gruppe umfassend potentiometrische, voltammetrische, amperometrische und optische Module.

4. Modulares System gemäss einem der Ansprüche 1 bis 3, **dadurch gekennzeichnet, dass** ein Messmodul als chemisch selektive Halbzelle ausgebildet ist und ein weiteres Modul als Referenzzelle (13).

5. Verfahren zur kontinuierlichen Bestimmung mindestens einer Substanz unter Verwendung eines modularen Systems nach einem der Ansprüche 1 bis 4, **dadurch gekennzeichnet, dass** mittels mindestens zweier Messmodule gleichen oder verschiedenen Modultyps mindestens eine Messgrösse ermittelt wird, wobei eine selektive Schichtstruktur je Modul einen selektiven Erkennungsschritt ermöglicht, dass der Erkennungsschritt mit mindestens einem Transduktionsschritt verbunden ist, der zur Bildung einer Messgrösse führt, dass die Messgrösse über mindestens eine Ableitvorrichtung abgeleitet wird und dass die Ableitung der Messgrösse zu mindestens einem Teil der Information über die Substanz führt.

6. Verfahren gemäss Anspruch 5, **dadurch gekennzeichnet, dass** mindestens eine Ableitvorrichtung mindestens eine Messgrösse aus einem Messmodul oder eine Bezugsgrösse aus dem Referenzmodul auf eine Vorrichtung zur Verarbeitung der Messgrössen ableitet, derart, dass die resultierende Information zur Prozesssteuerung verwendet werden kann.

7. Verwendung des Systems nach einem der Ansprüche 1 bis 4 zur Bestimmung anorganischer und organischer Ionen, geladener, ungeladener oder neutraler Moleküle, Salze, Isotope, Radikale, Zellen oder Zellbestandteile, Organismen, Mikroorganismen, Viren, Organellen, oder Rezeptoren.

8. Verwendung gemäss Anspruch 7 in der medizinischen Diagnostik und Behandlung, in der Dialyse und Haemodialyse, und in der klinischen Analytik.

9. Verwendung gemäss Anspruch 7 in der Umwelttechnik, Bautechnik, Reinraumtechnik, Abwasser-, Grund- und Trinkwasserkontrolle, in der Textiltechnik und in der Waschmittel- und Reinigungs- und Tensidtechnologie.

10. Verwendung gemäss Anspruch 7 in der Lebensmitteltechnologie und -produktion, in der Agrartechnologie, insbesondere in der Hors-sol-Kultur-Technik, in der Lebensmitteltechnologie, in der Biotechnologie und der chemischen Prozesstechnik, in der Pharmaforschung und -produktion, der Arzneimitteltechnologie und der Arzneimittelkontrolle.

11. Modul mit einer auswechselbaren selektiven Schichtstruktur (8), einem (er) Ableitvorrichtung/Ableitelement (12) und einem Körper (14) der Ableitvorrichtung/des Ableitelements (12) **dadurch gekennzeichnet, dass** das Modul einen Kanal (9) für den Durchfluss der Probe enthält, derart, dass das Innere des Probekanals (9) mit der selektiven Schichtstruktur (8) des jeweiligen Moduls in direktem Kontakt steht, und dass die Dichtung zur selektiven Schichtstruktur vor allem über die Elastizität der Schichtstruktur erfolgt.

12. Modul gemäss Anspruch 11, **dadurch gekennzeichnet, dass** es eine von der Ableitvorrichtung (12) getrennt vorliegende Schichtstruktur (8) aufweist und dass auch die Ableitvorrichtung (12) zusammen mit oder separat von dessen Körper (14) auswechselbar ist.

13. Modul gemäss Anspruch 11 oder 12, **dadurch gekennzeichnet, dass** es einen Probenkanal (9) aufweist, der mindestens zonenweise aus der Gegenelektrode besteht.

14. Modul gemäss Anspruch 11 oder 12, **dadurch gekennzeichnet, dass** es ein optisches Modul ist.

15. Modul gemäss Anspruch 14, **dadurch gekennzeichnet, dass** die selektive Schichtstruktur (8) mindestens einen Liganden ausgewählt aus der Gruppe bestehend aus N,N-disubstituierten aminophenyl-4'-trifluoroacetylazobenzolen, N,N-disubstituierten-aminonaphthalin-4'-trifluoroacetyl-azobenzolen, 4-Trifluoracetyl-4'-(N,N-disubstituerte-amino)stilbene und disubstituiertes p-amino-trifluoracetylstilbene enthält.

16. Modul gemäss Anspruch 14 oder 15, **dadurch gekennzeichnet, dass** die selektive Schichtstruktur mindestens einen Komplex eines 4,7-Diphenyl-1,10-phenanthrolins mit Ruthenium oder Lanthaniden enthält, der am Phenylrest lipophil substituiert ist, besonders in 4'-Position mit einem Alkylrest, vorzugsweise einem Propyl- bis Dodecyl-, insbesondere einem Propyl- bis Heptylrest.

17. Verwendung von symmetrischen Membranen und einer symmetrischen Anordnung/Konfiguration der Messzelle für kalibrationsfreie Messungen in Systemen nach einem der Ansprüche 1 bis 4.

18. Verwendung von symmetrischen Membranen und einer symmetrischen Anordnung/Konfiguration der Messzelle in Systemen nach einem der Ansprüche 1 bis 4 zur Herstellung von Referenzmaterialien für die Bestimmung der molalen Aktivität von Ionen zur Qualitätssicherung.

19. Verwendung von N,N-disubstiuierten-aminophenyl-4'-trifluoracetyl-azobenzolen, N,N-disubstituiertenaminonaphthalin-4'-trifluoracetyl-azobenzolen, 4-Trifluoracetyl-4'-(N,N-disubstituierten-amino)stilbenen und disubstituierten-p-amino-trifluoracetylstilbenen, wobei die Substituenten lipophil sind, als chromogene Liganden in Modulen nach einem der Ansprüche 1 bis 4 und 11 bis 16.

20. Verwendung eines Komplexes eines am Phenylrest lipophil substituierten 4,7-Diphenyl-1,10-phenanthrolins mit Ruthenium oder Lanthaniden zur optischen Sauerstoffbestimmung in Messystemen resp. Modulen nach einem der Ansprüche 1 bis 4 und 11 bis 16.

21. Verwendung eines Moduls nach einem der Ansprüche 11 bis 13, das eine enzym- und mediatorhaltige Paste aufweist, zur amperometrischen Bestimmung von Phosphat.

## Claims

1. Modular system for the continuous reagent-less determination of at least one substance, **characterized in that** it has at least two measuring modules (I, II, III) of the same or different modular type for the simultaneous yield of at least two informations in flow-through, whereby each module contains at least one exchangeable selective layered structure (8) and a transducing device (12), whereby the layered structure (8) enables a selective recognition step and allows for a continuous measuring, whereby said module contains a channel (9) for the flow-through of the sample, such that the interior of said sample channel (9) is in direct contact with said selective layered structure (8) of said module, and whereby the sealing to the selective layered structure primarily succeeds by the elasticity of the layered structure.

2. Modular system according to claim 1, **characterized in that** it is multidimensional, i.e. that it comprises measuring modules (I, II, III) of at least two modular types.

3. Modular system according to claim 1 or 2, **characterized in that** the measuring modules are selected from the group comprising potentiometric, voltammetric, amperometric and optical modules.

4. Modular system according to one of claims 1 to 3, **characterized in that** a measuring module is provided as a chemically selective half-cell and a further module as reference-cell (13).

5. Process for the continuous determination of at least one substance by using a modular system according to one of claims 1 to 4, **characterized in that** through at least two measuring modules of the same or different modular type at least one measured parameter is determined, whereby a selective layered structure per module allows for a selective recognition step, said recognition step is associated with at least one transduction step leading to the formation of a measured parameter, said measured parameter is transduced through at least one transduction device and said transduction of the measured parameter leads to at least part of the information concerning the substance.

6. Process according to claim 5, **characterized in that** at least one transducing device transduces at least one measured parameter from a measuring module or a reference parameter from the reference module onto a device for the transformation of the measured parameters, such that the resulting information can be used for process control.

7. Use of the system according to one of claims 1 to 4 for the determination of inorganic and organic ions, charged, uncharged and neutral molecules, salts, isotopes, radicals, cells or cell components, organisms, micro-organisms, viruses, organellae, or receptors.

8. Use according to claim 7 in medical diagnostics and treatment, in dialysis and hemodialysis and in clinical analytics.

9. Use according to claim 7 in the environmental technology, building technology, clean-room technology, waste water-, ground- and drinking-water surveillance, in the textile technology and in the detergent and cleaning and surfactant technology.

10. Use according to claim 7 in the food technology and production, in the agriculture, notably in soilless cultures, in the food technology, in biotechnology, and in the chemical process control, in the pharmaceutical research and production, the drug technology and the drug control.

11. Module with an exchangeable selective layered structure (8), a transducing device/transducing element (12) and a body (14)of the transducing device/transducing element (12), **characterized in that** said module contains a channel (9) for the flow-through of the sample, such that the interior of said sample channel (9) is in direct contact with said selective layered structure (8) of said module, and whereby the sealing to the selective layered structure primarily succeeds by the elasticity of the layered structure.

12. Module according to claim 11, **characterized in that** it has a layered structure (8) being separated from the transducing device (12) and that also the transducing device (12) is exchangeable together or separately from its body (14).

13. Module according to claim 11 or 12, **characterized in that** it has a sample channel (9), consisting at least partially of the counter-electrode.

14. Module according to claim 11 or 12, **characterized in that** it is an optical module.

15. Module according to claim 14, **characterized in that** the selective layered structure (8) contains at least one ligand selected from the group consisting of N,N-disubstituted aminophenyl-4'-trifluoroacetyl-azobenzenes, N,N-disubstituted aminonaphtalene-4'-trifluoroacetyl-azobenzenes, 4-trifluoroacetyl-4'-(N,N-disubstituted amino)stilbenes and disubstituted p-amino-trifluoroacetylstilbenes.

16. Module according to claim 14 or 15, **characterized in that** the selective layered structure contains at least one complex of a 4,7-diphenyl-1,10-phenanthrolene with ruthenium or lanthanides which is lipophilically substituted at the phenyl moiety, especially in 4'-position with an alkyl group, preferably a propyl to dodecyl group, especially a propyl to heptyl group.

17. Use of symmetrical membranes and a symmetrical arrangement/configuration of the measuring cell for calibration-free measurements in systems according to one of claims 1 to 4.

18. Use of symmetrical membranes and of a symmetrical arrangement/configuration of the measuring cell within systems according to one of claims 1 to 4 for the preparation of reference materials for the determination of molal activities of ions for quality assessment.

19. Use of N,N-disubstituted-aminophenyl-4'-trifluoroacetyl-azobenzenes, N,N-disubstituted aminonaphtalene-4'-trifluoroacetyl-azobenzenes, 4-trifluoroacetyl-4'-(N,N-disubstituted-amino)stilbenes and disubstituted-p-amino-trifluoracetylstilbenes, whereby the substituents are lipohilic, as chromogenic ligands in modules according to one of claims 1 to 4 and 11 to 16.

20. Use of a complex of a 4,7-diphenyl-1,10-phenanthrolene with ruthenium or lanthanides, lipophilically substituted at the phenyl moiety, for the optical determination of oxygen in a measuring system or module, respectively, according to one of claims 1 to 4 and 11 to 16.

21. Use of a module according to one of claims 11 to 13 which has an enzyme and mediator-containing paste for the amperometric determination of phosphate.

## Revendications

1. Système modulaire pour la détermination en continu sans réactif d'au moins une substance,
**caractérisé en ce qu'**il comporte au moins deux modules de mesure (I, II, III) de type identique ou différent pour l'obtention l'exploitation simultanée d'au moins deux informations dans le flux, chaque module contenant au moins une structure en couche (8) sélective interchangeable et un dispositif de dérivation (12), la structure en couche (8) permettant une étape de reconnaissance sélective et autorisant une mesure continue, le module comportant un canal (9) pour le passage de l'échantillon de sorte que l'intérieur du canal d'échantillon (9) se trouve en contact direct avec la structure en couche sélective (8) de chaque module, et que l'étanchéité en regard de la structure en couche sélective soit assurée principalement au moyen de l'élasticité de la structure en couche.

2. Système modulaire selon la revendication 1,
**caractérisé en ce qu'**il est multidimensionnel, c'est-à-dire que le module de mesure (I, II, III) comprend au moins deux types de modules.

3. Système modulaire selon la revendication 1 ou 2,
**caractérisé en ce que** les modules de mesure sont choisis dans le groupe comprenant les modules potentiométrique, voltamétrique, ampèrométrique et optiques.

4. Système modulaire selon l'une des revendications 1 à 3,
**caractérisé en ce qu'**un module de mesure est réalisé sous forme d'une demi-cellule chimique sélective et un autre module sous forme d'une cellule de référence (13).

5. Procédé pour déterminer en continu au moins une substance par utilisation d'un système modulaire selon l'une des revendications 1 à 4,
**caractérisé en ce que**, à l'aide d'au moins deux modules de mesure de type identique ou différent, au moins une grandeur de mesure est obtenue, une structure en couche sélective rendant possible pour chaque module une étape de reconnaissance sélective, **en ce que** l'étape de reconnaissance est reliée à au moins une étape de transduction qui conduit à la formation d'une grandeur de mesure, **en ce que** la grandeur de mesure est dérivée au moyen d'au moins un dispositif de dérivation et **en ce que** la dérivation de la grandeur de mesure conduit à au moins une partie de l'information sur la substance.

6. Procédé selon la revendication 5,
**caractérisé en ce que**, au moins un dispositif de dérivation dérive au moins une grandeur de mesure à partir d'un module de mesure ou bien une grandeur de référence à partir du module de référence, vers un dispositif de traitement des grandeurs de mesure de sorte que l'information résultante peut être utilisée pour la commande de processus.

7. Utilisation du système selon l'une des revendications 1 à 4 pour la détermination d'ions non organiques et organiques, de molécules chargées, non chargées ou neutres, de sels, d'isotopes, de radicaux, de cellules ou de composants de cellules, d'organismes, de microorganismes, de virus, d'organes ou de récepteurs.

8. Utilisation selon la revendication 7 dans le diagnostic et le traitement médical, en dialyse et hémodialyse et en analytique clinique.

9. Utilisation selon la revendication 7 dans le domaine écologique, les techniques de construction, la technique de la salle blanche, le contrôle des eaux usées, du sol et de l'eau potable, l'industrie du textile et les industries des agents de lavage, de nettoyage et de dérivés tensio-actifs.

10. Utilisation selon la revendication 7 dans la technologie et la production de denrées alimentaires, la technologie agricole, en particulier dans la technique de culture hors sol, dans la technologie des produits alimentaires, dans la biotechnologie et dans la technique des processus chimiques, dans la recherche et la production pharmaceutiques, dans la technologie des médicaments et dans le contrôle des médicaments.

11. Module avec une structure en couches sélective interchangeable (8), un dispositif/élément de dérivation (12) et un corps (14) du dispositif/élément de dérivation **caractérisé en ce que** le module comprend un canal (9) pour le passage de l'échantillon de sorte que l'intérieur du canal pour échantillon se trouve en contact direct avec la structure en couche sélective (8) du module concerné et **en ce que** l'étanchéité vers la structure en couche sélective est assurée principalement par de l'élasticité de la structure en couche.

12. Module selon la revendication 11,
**caractérisé en ce qu'**il présente une structure en couche (8) se trouvant en avant et séparée du dispositif de dérivation (12) et **en ce que** le dispositif de dérivation (12) peut aussi être échangé simultanément au corps (14) ou séparément.

13. Module selon la revendication 11 ou 12,
**caractérisé en ce qu'**il comporte un canal pour échantillon (9) qui est constitué au moins par endroits par la contre électrode.

14. Module selon la revendication 11 ou 12,
**caractérisé en ce que** c'est un module optique.

15. Module selon la revendication 14,
**caractérisé en ce que** la structure en couche sélective (8) contient au moins un ligand choisi parmi le groupe constitué des N,N-disubstitué aminophényl-4'-trifluoroacetyl-azobenzols, N,N-disubstitué aminonaphtaline-4'-trifluoroacetyl-azobenzols, 4-trifluoroacetyl-4'-(N,N-disubstitué-amino)stilbene et disubstitué p-amino-trifluoroacetylstilbene.

16. Module selon la revendication 14 ou 15,
**caractérisé en ce que** la structure en couche sélective comprend au moins un complexe d'une 4,7-diphényl-1,10-phenanthroline avec du Ruthénium ou des lanthanides qui est substitué sur le radical phényle lipophile, en particulier en position 4' avec un radical alkyle, de préférence un radical propyle à dodécyle, en particulier un radical propyle à heptyle.

17. Utilisation de membranes symétriques et d'une configuration/agencement symétrique des cellules de mesure pour des mesures sans calibrage dans des systèmes selon l'une des revendications 1 à 4.

18. Utilisation de membranes symétriques et d'un agencement/configuration symétrique des cellules de mesure dans des systèmes selon l'une des revendications 1 à 4, pour la fabrication de matériaux de référence pour la détermination de l'activité molale d'ions pour la garantie de la qualité.

19. Utilisation du N,N-disubstitué aminophényl-4'-trifluoroacetylazobenzol, des N,N-disubstitué aminonaphtaline-4'-trifluoroacetylazobenzols, du 4-trifluoroacetyl-4'-(N,N-disubstitué-amino)stilbene et du disubstitué p-amino-trifluoroacetylstilbene, les substituants lipophiles étant, dans les modules selon l'une des revendications 1 à 4 et 11 à 16, sous forme de ligands chromogènes.

20. Utilisation d'un complexe d'un 4,7-diphényl-1,10-phenanthroline avec du Ruthénium ou des lanthanides substitué sur le radical phényle lipophile pour la détermination optique d'oxygène dans les systèmes de mesure ou modules selon l'une des revendications 1 à 4 et 11 à 16.

21. Utilisation d'un module selon l'une des revendications 11 à 13, qui comporte une pâte contenant une enzyme ou un médiateur pour la détermination ampérométrique de phosphate.
